# EUROPÄISCHE PATENTANMELDUNG

(11) **EP 0 591 806 A1**
(43) Veröffentlichungstag der Anmeldung: **13.04.1994**
(21) Anmeldenummer: 93115563.4
(22) Anmeldetag: 27.09.1993
(51) Int. Cl.: C07D 207/36, A01N 43/36

(54) **Substituierte 2-Arylpyrrole**

(30) Priorität: 08.10.1992 DE 4233885
(71) Anmelder: BAYER AG, 51368 Leverkusen (DE)
(72) Erfinder: Uhr, Hermann Dr., D-51379 Leverkusen (DE); Marhold, Albrecht Dr., D-51373 Leverkusen (DE); Böhm, Stefan Dr., D-51065 Köln (DE); Erdelen, Christoph Dr., D-42799 Leichlingen (DE); Wachendorff-Neumann, Ulrike Dr., D-40789 Monheim (DE); Stendel, Wilhelm Dr., D-42113 Wuppertal (DE)

(57) **Zusammenfassung**

Die vorliegende Erfindung betrifft neue substituierte 2-Arylpyrrole der Formel (I) 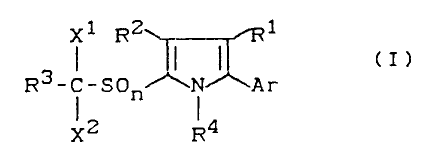
in welcher
- n: für 0, 1 oder 2 steht,
- Ar: für gegebenenfalls substituiertes Aryl steht,
- R¹ und R²: unabhängig voneinander für Wasserstoff oder Halogen stehen, wobei jedoch mindestens einer der beiden Reste R¹ oder R² für Halogen steht,
- R³: für Wasserstoff, Halogen oder gegebenenfalls substituiertes Alkyl steht,
- X¹ und X²: unabhängig für Wasserstoff oder Halogen stehen und
- R⁴: für Wasserstoff oder 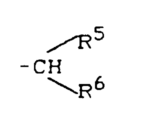 steht,
(wobei R⁵ und R⁶ die in der Beschreibung angegebenen Bedeutungen haben),
ferner Verfahren und neue Zwischenprodukte zu ihrer Herstellung sowie ihre Verwendung als Schädlingsbekämpfungsmittel.

## Beschreibung

Die vorliegende Erfindung betrifft neue substituierte 2-Arylpyrrole, Verfahren und neue Zwischenprodukte zu ihrer Herstellung und ihre Verwendung zur Bekämpfung von tierischen Schädlingen, insbesondere von Insekten, Spinnentieren und Nematoden, die in der Landwirtschaft, in den Forsten, in Vorrats- und Materialschutz sowie auf dem Hygienesektor vorkommen.

Es ist bereits bekannt geworden, daß strukturell ähnliche Cyanopyrrole als Molluskizide, Fungizide und Insektizide wirksam sind (siehe dazu z.B. EP-A 0 347 488, EP-A 0 358 047, EP-A 0 312 723, DE-A 4 117 752, EP-A 0 481 182, EP-A 0 484 614). Die Wirksamkeit und Wirkungsbreite dieser Verbindungen ist jedoch insbesondere bei niedrigen Aufwandmengen und Konzentrationen nicht immer voll zufriedenstellend.

Es wurden nun neue substituierte 2-Arylpyrrole der allgemeinen Formel (I) gefunden, 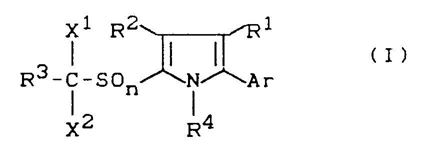
in welcher
- R¹ und R²: unabhängig voneinander für Wasserstoff oder Halogen stehen, wobei jedoch mindestens einer der beiden Reste R¹ oder R² für Halogen steht,
- R³: für Wasserstoff, Halogen oder gegebenenfalls substituiertes Alkyl steht,
- R⁴: für Wasserstoff oder 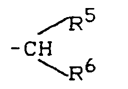 steht,
worin R⁵ für Wasserstoff oder gegebenenfalls substituiertes Alkyl steht und R⁶ für Wasserstoff, gegebenenfalls substituiertes Alkyl oder für einen der Reste 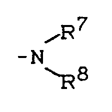
oder -O-R⁷ steht,
worin R⁷ und R⁸ unabhängig voneinander für Wasserstoff, Alkyl, Alkenyl, Alkinyl, Alkoxycarbonyl, Alkenoxycarbonyl, Alkinoxycarbonyl oder Acyl stehen, wobei R⁷ und R⁸ gemeinsam mit dem N-Atom, an welches sie gebunden sind, auch einen Ring bilden können,
- Ar: für gegebenenfalls substituiertes Aryl steht,
- X¹ und X²: unabhängig voneinander für Wasserstoff oder Halogen stehen, und
- n: für 0, 1 oder 2 steht.

Weiterhin wurde gefunden, daß man die substituierten 2-Arylpyrrole (I) - in Abhängigkeit von den jeweiligen Substituenten - auf unterschiedlichen Verfahrenswegen herstellen kann, wobei bestimmte Untergruppen von Endprodukten der Formel (I) zugleich auch Zwischenprodukte bei der Herstellung anderer Endprodukte (I) sein können; eine Übersicht zeigt das folgende Schema 1: 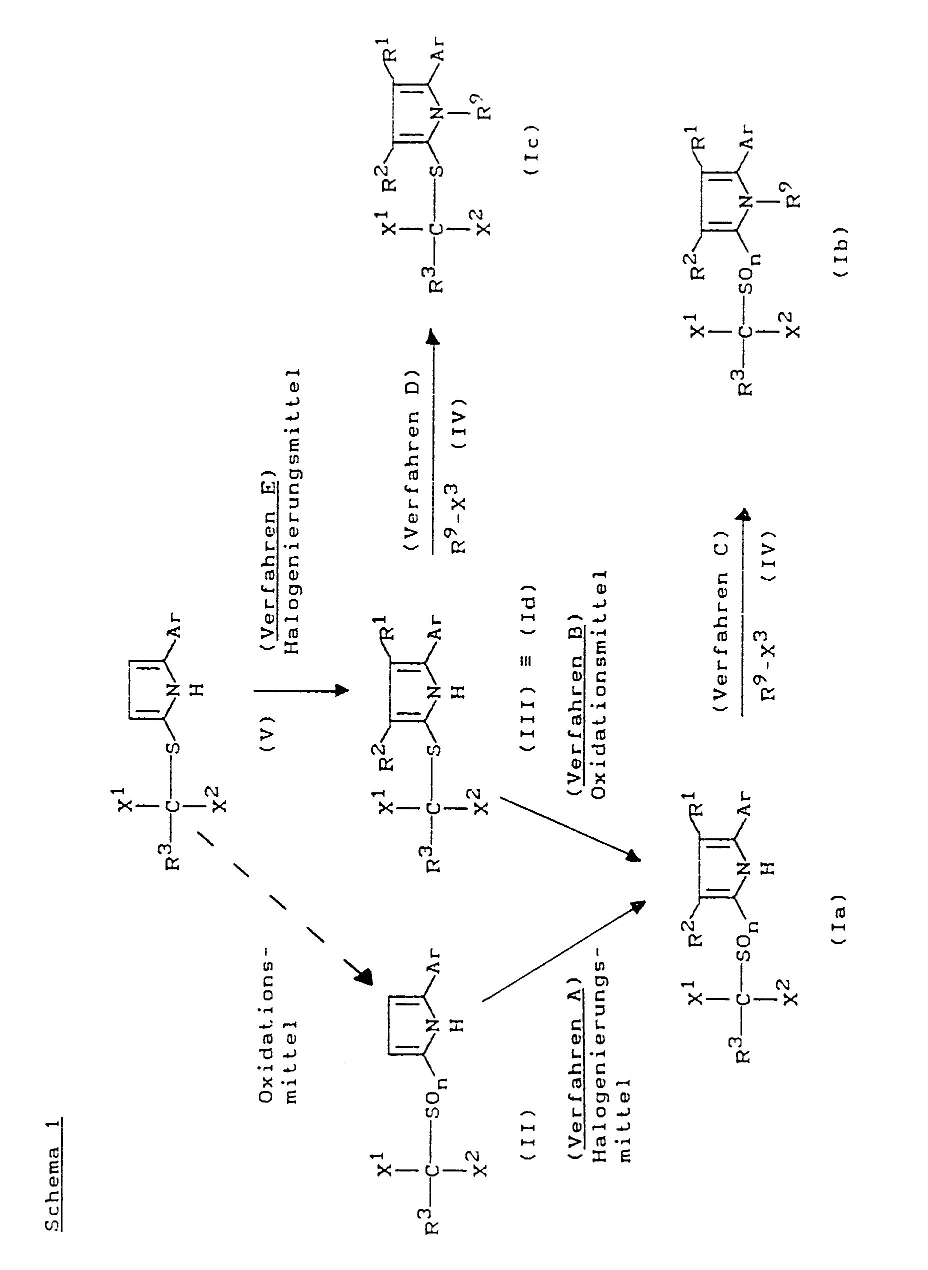
So erhält man die substituierten 2-Arylpyrrole der allgemeinen Formel (Ia), 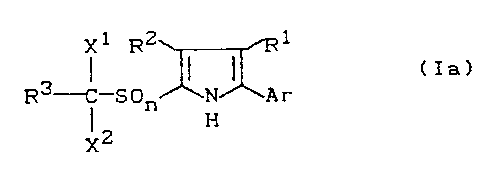
in welcher R¹, R², R³, X¹, X² und Ar die oben angegebene Bedeutung haben und n = 1 oder 2 ist,
wenn man entweder
A) 2-Arylpyrrole der Formel (II) 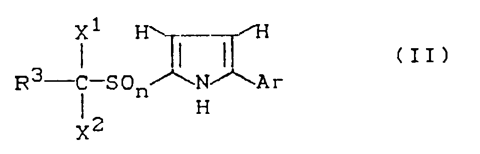 in welcher R³, X¹, X² und Ar die oben angegebenen Bedeutungen haben und n = 1 oder 2 ist,
   mit Halogenierungsmitteln umsetzt (Verfahren A)
   oder
B) 2-Arylpyrrole der Formel (III) 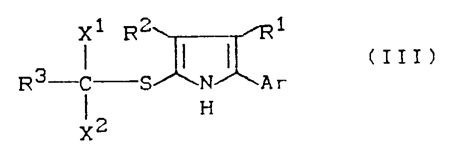 in welcher R¹, R², R³, X¹, X² und Ar die oben angegebenen Bedeutungen haben,
   mit Oxidationsmitteln umsetzt (Verfahren B).
C) Man erhält 2-Arylpyrrole der Formel (Ib),

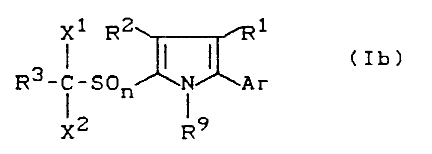

in welcher R¹, R², R³, X¹, X² und Ar die oben angegebene Bedeutung haben und n = 1 oder 2 ist und
- R⁹: für 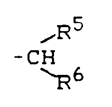 steht, worin R⁵ und R⁶ die oben angegebene Bedeutung haben,
wenn man 2-Arylpyrrole der allgemeinen Formel (Ia), in welcher R¹, R², R³ X¹, X² und Ar die oben angegebene Bedeutung haben und n = 1 oder 2 ist,
mit Verbindungen der Formel (IV)

R⁹-X³ (IV)

in welcher
R⁹ die oben angegebene Bedeutung hat und
X³ für eine anionische Abgangsgruppe steht,
gegebenenfalls in Gegenwart von Basen und/oder gegebenenfalls in Gegenwart von Verdünnungsmitteln umsetzt (Verfahren C).
D) Weiterhin erhält man 2-Arylpyrrole der Formel (Ic),

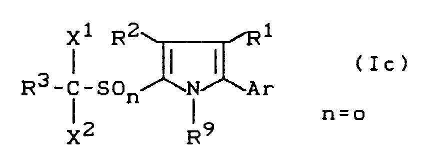

in der R¹, R², R³, X¹, X² und Ar die oben angegebene Bedeutung haben und
- R⁹: für 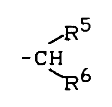 steht, wobei R⁵ und R⁶
die oben angegebene Bedeutung haben und n = 0 ist,
wenn man 2-Arylpyrrole der allgemeinen Formel (III), in welcher R¹, R², R³, X¹, X² und Ar die oben angegebene Bedeutung haben,
mit Verbindungen der Formel (IV)

R⁹-X³ (IV)

in welcher
R⁹ die oben angegebene Bedeutung hat und
X³ für eine anionische Abgangsgruppe steht,
gegebenenfalls in Gegenwart von Basen und/oder gegebenenfalls in Gegenwart von Verdünnungsmitteln umsetzt (Verfahren D).
E) Außerdem erhält man die substituierten 2-Arylpyrrole der allgemeinen Formel (Id),

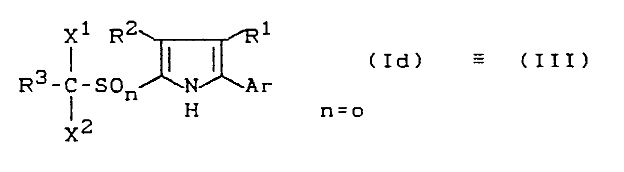

in welcher R¹, R², R³, X¹, X² und Ar die oben angegebene Bedeutung haben und n = 0 ist,
wenn man 2-Arylpyrrole der Formel (V) 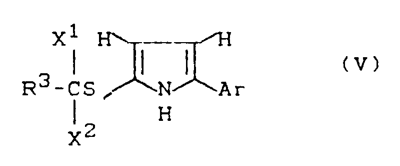
in welcher R³, X¹ , X² und Ar die oben angegebene Bedeutung haben,
mit Halogenierungsmitteln umsetzt (Verfahren E).
(Die substituierten 2-Arylpyrrole der allgemeinen Formel (Id) sind identisch mit den durch die allgemeine Formel (III) beschriebenen Vorprodukten).

Schließlich wurde gefunden, daß die neuen substituierten 2-Arylpyrrole der Formel (I) stark ausgeprägte biologische Eigenschaften besitzen und vor allem zur Bekämpfung von tierischen Schädlingen, insbesondere von Insekten, Spinnentieren und Nematoden, die in der Landwirtschaft, in den Forsten, im Vorrats- und Materialschutz sowie auf dem Hygienesektor vorkommen, geeignet sind. Außerdem eignen sich die neuen substituierten 2-Arylpyrrole der Formel (I) auch zur Bekämpfung pflanzenpathogener Pilze.

Bevorzugt sind substituierte 2-Arylpyrrole der obigen Formel (I), in welcher
R¹ und R² unabhängig voneinander für Wasserstoff, Brom oder Chlor stehen, wobei mindestens einer der Reste R¹ oder R² für Brom oder Chlor steht,
R³ für Wasserstoff, Fluor, Chlor, Brom oder C₁-C₅-Alkyl steht (welches gegebenenfalls gleich oder ungleich substituiert ist durch 1 bis 5 Fluor-, Chlor- oder Bromatome),
R⁴ für Wasserstoff oder 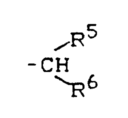
steht,
worin R⁵ für Wasserstoff oder C₁-C₅-Alkyl steht (welches gegebenenfalls substituiert ist durch 1 bis 5 gleiche oder verschiedene Halogenatome, durch C₁-C₅-Alkoxy, C₁-C₅-Alkylthio, C₁-C₅-Acyloxy, C₂-C₆-Alkoxycarbonyl, Phenyl, Cyano oder durch Nitro) und
R⁶ für Wasserstoff oder C₁-C₆-Alkyl steht (welches gegebenenfalls substituiert ist durch 1 bis 5 gleiche oder verschiedene Halogenatome, durch C₁-C₈-Alkoxy, C₁-C₈-Alkylthio, C₁-C₆-Acyloxy, C₂-C₈-Alkoxycarbonyl, Phenyl, Cyano oder durch Nitro) oder worin R⁶ für 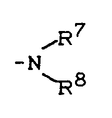
oder -O-R⁷ steht,
wobei R⁷ und R⁸ unabhängig voneinander für Wasserstoff, C₁-C₈-Alkyl, C₃-C₈-Alkenyl oder C₃-C₈-Alkinyl stehen (wobei die Alkyl-, Alkenyl- und Alkinylreste jeweils gegebenenfalls durch 1 bis 6 gleiche oder verschiedene Halogenatome, C₁-C₆-Alkoxy, C₁-C₆-Alkylthio, C₁-C₆-Acyloxy, (C₁-C₆-Alkoxy)-carbonyl, gegebenenfalls substituiertes Phenyl, Cyano oder Nitro substituiert sind), oder für (C₁-C₈-Alkoxy)-carbonyl, (C₃-C₈-Alkenoxy)-carbonyl oder (C₃-C₈-Alkinoxy)-carbonyl stehen (wobei die Alkoxy-, Alkenoxy- und Alkinoxyreste jeweils gegebenenfalls durch 1 bis 6 gleiche oder verschiedene Halogenatome, C₁-C₆-Alkoxy, C₁-C₆-Alkylthio, C₁-C₆-Acyloxy, (C₁-C₆-Alkoxy)-carbonyl, gegebenenfalls substituiertes Phenyl, Cyano oder Nitro substituiert sind), oder für C₁-C₈-Acyl stehen (welches gegebenenfalls durch 1 bis 6 gleiche oder verschiedene Halogenatome, C₁-C₆-Alkoxy, C₁-C₆-Alkylthio, C₁-C₆-Acyloxy, (C₁-C₆-Alkoxy)-carbonyl, gegebenenfalls substituiertes Phenyl, Cyano oder Nitro substituiert ist), oder worin R⁷ und R⁸ zusammen mit dem N-Atom, an welches sie gebunden sind, einen 4- bis 8-gliedrigen Ring bilden,
Ar für Phenyl steht, welches gegebenenfalls ein- bis fünffach gleich oder verschieden substituiert ist durch Halogen,
durch C₁-C₈-Alkyl, C₂-C₈-Alkenyl oder C₂-C₈-Alkinyl (wobei die Alkyl-, Alkenyl- und Alkinylreste jeweils gegebenenfalls durch 1 bis 6 Halogenatome, C₁-C₅-Alkoxy, C₁-C₅-Alkylthio oder durch C₁-C₅-Acyloxy substituiert sind, und wobei die Alkoxy- und Alkylthio-Reste jeweils durch 1-6 Halogenatome substituiert sein können),
durch C₁-C₈-Alkoxy, C₂-C₈-Alkenoxy oder C₂-C₈-Alkinoxy (wobei die Alkoxy-, Alkenoxy- und Alkinoxyreste gegebenenfalls durch 1 bis 6 Halogenatome substituiert sind),
durch C₁-C₈-Alkylthio, C₂-C₈-Alkenylthio oder C₂-C₈-Alkinylthio (wobei die Alkylthio-, Alkenylthio- und Alkinylthio-Reste jeweils gegebenenfalls durch 1 bis 6 Halogenatome substituiert sind),
durch C₂-C₈-Acyloxy (welches gegebenenfalls durch 1 bis 6 Halogenatome substituiert ist),
durch Amino (welches gegebenenfalls substituiert ist durch 1 bis 2 gleiche oder verschiedene Alkylreste oder Halogenalkylreste mit 1 bis 8 Kohlenstoffatomen und gegebenenfalls 1 bis 6 Halogenatomen),
durch Nitro oder Cyano,
und worin
X¹ und X² unabhängig voneinander für Wasserstoff, Fluor, Chlor oder Brom stehen und
n für 0, 1 oder 2 steht.

Besonders bevorzugt sind substituierte 2-Arylpyrrole der Formel (I), in welcher
R¹ und R² unabhängig voneinander für Wasserstoff, Brom oder Chlor stehen, wobei mindestens einer der beiden Reste R¹ oder R² für Brom oder Chlor steht,
R³ für Wasserstoff, Fluor, Chlor, Brom oder C₁-C₄-Alkyl steht (welches gegebenenfalls gleich oder ungleich substituiert ist durch 1 bis 5 Fluor-, Chlor- oder Bromatome),
R⁴ für Wasserstoff oder 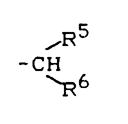
steht,
worin R⁵ für Wasserstoff oder C₁-C₄-Alkyl steht (welches gegebenenfalls substituiert ist durch 1 bis 5 gleiche oder verschiedene Halogenatome, durch C₁-C₄-Alkoxy, C₁-C₄-Alkylthio, C₁-C₄-Acyloxy, C₂-C₅-Alkoxycarbonyl, Phenyl, Cyano oder durch Nitro), und
R⁶ für Wasserstoff oder C₁-C₅-Alkyl steht (welches gegebenenfalls substituiert ist durch 1 bis 5 gleiche oder verschiedene Halogenatome, durch C₁-C₆-Alkoxy, C₁-C₆-Alkylthio, C₁-C₅-Acyloxy, C₂-C₆-Alkoxycarbonyl, Phenyl, Cyano oder durch Nitro), oder worin R⁶ für 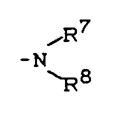
oder -O-R⁷ steht,
worin R⁷ und R⁸ unabhängig voneinander für Wasserstoff, C₁-C₆-Alkyl, C₃-C₆-Alkenyl oder C₃-C₆-Alkinyl stehen (wobei die Alkyl-, Alkenyl- und Alkinylreste jeweils gegebenenfalls durch 1 bis 5 Fluor- und/oder Chloratome, C₁-C₄-Alkoxy, C₁-C₄-Alkylthio, C₁-C₄-Acyloxy, (C₁-C₄-Alkoxy)-carbonyl, gegebenenfalls substituiertes Phenyl, Cyano oder Nitro substituiert sind),
oder für (C₁-C₆-Alkoxy)-carbonyl, (C₃-C₆-Alkenoxy)- oder für (C₃-C₆-Alkinoxy)-carbonyl stehen (wobei die Alkoxy-, Alkenoxy- und Alkinoxyreste jeweils gegebenenfalls durch 1 bis 5 Fluor- und/oder Chloratome, C₁-C₄-Alkoxy, C₁-C₄-Alkylthio, C₁-C₄-Acyloxy, (C₁-C₄-Alkoxy)-carbonyl, gegebenenfalls substituiertes Phenyl, Cyano oder Nitro substituiert sind),
oder für C₁-C₆-Acyl stehen (welches gegebenenfalls durch 1 bis 5 Fluor- und/oder Chloratome, C₁-C₄-Alkoxy, C₁-C₄-Alkylthio, C₁-C₄-Acyloxy, (C₁-C₄-Alkoxy)-carbonyl, gegebenenfalls substituiertes Phenyl, Cyano oder durch Nitro substituiert ist),
oder R⁷ und R⁸ zusammen mit dem N-Atom, an welches sie gebunden sind, über eine beliebige Stelle zu einem 4-bis 6-gliedrigen Ring verknüpft sein können;
in welcher ferner
Ar für Phenyl steht, welches gegebenenfalls ein- bis vierfach gleich oder verschieden substituiert ist durch Fluor, Chlor oder Brom, oder
durch C₁-C₆-Alkyl, C₂-C₆-Alkenyl oder C₂-C₆-Alkinyl (wobei die Alkyl-, Alkenyl- und Alkinylreste jeweils gegebenenfalls durch 1 bis 5 Fluor- und/oder Chloratome, C₁-C₄-Alkoxy, C₁-C₄-Alkylthio oder durch C₁-C₄-Acyloxy substituiert sind, und wobei die Alkoxy- und Alkylthioreste jeweils durch 1 bis 5 Fluor- und/oder Chloratome substituiert sein können),
durch C₁-C₆-Alkoxy, C₂-C₆-Alyenoxy oder C₂-C₆-Alkinoxy (wobei die Alkoxy-, Alkenoxy- und Alkinoxyreste gegebenenfalls durch 1 bis 5 Fluor- und/oder Chloratome substituiert sind),
durch C₁-C₆-Alkylthio, C₂-C₆-Alkenylthio oder C₂-C₆-Alkinylthio (wobei die Alkylthio-, Alkenylthio- und Alkinylthioreste gegebenenfalls durch 1 bis 5 Fluor- und/oder Chloratome substituiert sind),
durch C₂-C₆-Acyloxy (welches gegebenenfalls durch 1 bis 5 Fluor- und/oder Chloratome substituiert ist),
durch Amino (welches gegebenenfalls substituiert ist durch 1 bis 2 gleiche oder verschiedene Alkylreste mit 1 bis 6 Kohlenstoffatomen, welche durch 1 bis 5 Fluor- und/oder Chloratome substituiert sein können),
durch Nitro oder Cyano,
und worin
X¹ und X² unabhängig voneinander für Wasserstoff, Fluor oder Chlor stehen und
n für 0, 1 oder 2 steht.

Die oben aufgeführten allgemeinen oder in Vorzugsbereichen aufgeführten Restedefinitionen bzw. Erläuterungen galten für die Endprodukte und für die Ausgangs- und Zwischenprodukte entsprechend. Diese Restedefinitionen können untereinander, also auch zwischen den jeweiligen Vorzugsbereichen beliebig kombiniert werden.

Verwendet man gemäß Herstellungsverfahren A 2- (4-Chlorphenyl)-5-(trifluormethylsulfonyl)-pyrrol und Brom als Ausgangsstoffe, so kann der Reaktionsverlauf durch das folgende Reaktionsschema wiedergegeben werden: 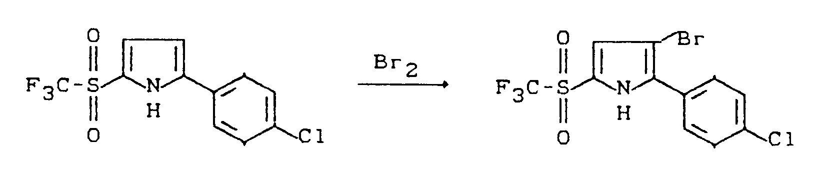
Verwendet man gemaß Verfahren B 2-(4-Chlorphenyl)-3,4-dibrom-5(trifluormethylthio)-pyrrol und m-Chlorperbenzoesaure als Ausgangsstoffe, so kann der Reaktionsverlauf durch das folgende Reaktionsschema wiedergegeben werden: 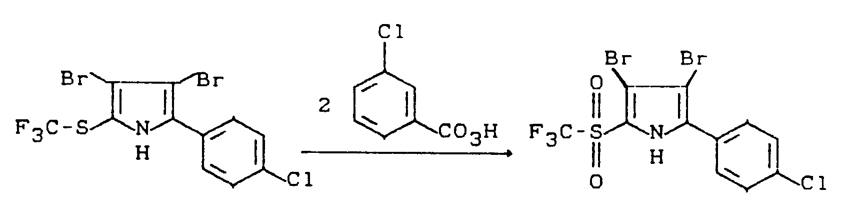
Verwendet man gemäß Verfahren C 2-(3,4-Dichlorphenyl)-3-brom-5-(chlordifluormethylsulfonyl)-pyrrol und (Chlormethyl)ethylether als Ausgangsstoffe, so kann der Reaktionsverlauf durch das folgende Reaktionsschema wiedergegeben werden: 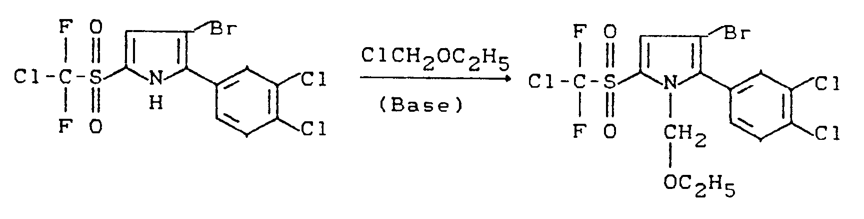
Verwendet man gemäß Verfahren D 2-(4-Chlorphenyl)-3,4-dibrom-5-(difluorchlormethylthio)-pyrrol und (Chlormethyl)ethylether als Ausgangsstoffe, so kann der Reaktionsverlauf durch das folgende Reaktionsschema wiedergegeben werden: 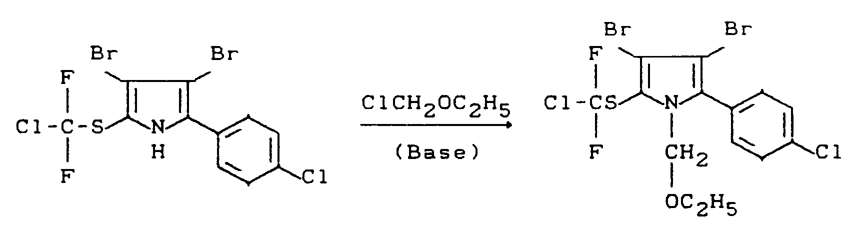
Verwendet man gemäß Verfahren E 2-(4-Chlorphenyl)-5-(difluorchlormethylthio)-pyrrol und Brom als Ausgangsstoffe, so kann der Reaktionsverlauf durch das folgende Reaktionsschema wiedergegeben werden: 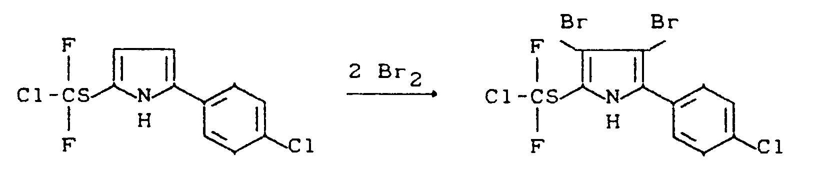
Das Verfahren A zur Herstellung von Verbindungen der Formel (Ia) ist dadurch gekennzeichnet, daß man 2-Arylpyrrole der Formel (II) mit Halogenierungsmitteln behandelt.

Als Verdünnungsmittel können bei Verfahren A alle üblichen Lösungsmittel eingesetzt werden. Vorzugsweise verwendbar sind Kohlenwasserstoffe wie Benzol und Hexan, halogenierte Kohlenwasserstoffe wie Chloroform und Methylenchlorid, weiterhin Ether wie Dibutylether, Tetrahydrofuran, Dioxan, außerdem organische Säuren, wie Ameisensäure und Essigsäure. Weiterhin kann auch Wasser als Verdünnungsmittel eingesetzt werden.

Als Halogenierungsmittel können alle üblichen Halogenierungsmittel eingesetzt werden. Vorzugsweise verwendet man Chlor, Brom, Natriumhypochlorit, Kaliumhypochlorit, Natriumhypobromit, Kaliumhypobromit, Sulfurylchlorid, t-Butylhypochlorit, N-Chlorsuccinimid und N-Bromsuccinimid.

Die Temperaturen können innerhalb eines größeren Bereichs variiert werden, Im allgemeinen arbeitet man bei Temperaturen von -10°C bis +120°C, vorzugsweise zwischen 0°C und 70°C.

Bei der Durchführung setzt man die Reaktionskomponenten der Formel (II) mit äquimolaren oder überschüssigen Mengen eines Halogenierungsmittels um.

Diese Umsetzung wird im allgemeinen unter Normaldruck durchgeführt. Im Falle von Chlor und Brom kann die Reaktion auch bei erhöhtem Druck (bis 5000 hPa) durchgeführt werden.

Das Verfahren B zur Herstellung von Verbindungen der Formel (Ia) ist dadurch gekennzeichnet, daß man 2-Arylpyrrole der Formel (III) mit Oxidationsmitteln umsetzt.

Als Verdünnungsmittel können bei Verfahren B alle üblichen Lösungsmittel eingesetzt werden, Vorzugsweise verwendbar sind Kohlenwasserstoffe wie Benzol und Hexan, halogenierte Kohlenwasserstoffe wie Chloroform und Methylenchlorid, weiterhin Ether wie Dibutylether, Tetrahydrofuran, Dioxan, außerdem organische Sauren, wie Ameisensäure und Essigsäure, Weiterhin kann auch Wasser als Verdünnungsmittel eingesetzt werden.

Als Oxidationsmittel können alle üblichen Oxidationsmittel eingesetzt werden Vorzugsweise verwendet man m-Chlor-perbenzoesäure, Kaliumhydrogenperoxodisulfat (Oxone), Magnesiummonoperoxyphthalat, H₂O₂, Luftsauerstoff in Gegenwart von Katalysatoren, Kaliumpermanganat oder CrO₃.

Die Temperaturen können innerhalb eines größeren Bereichs variiert werden Im allgemeinen arbeitet man bei Temperaturen von -30°C bis +200°C, vorzugsweise zwischen -10°C und +80°C.

Bei der Durchführung setzt man die Reaktionskomponenten der Formel (III) je nach angestrebter Oxidationsstufe des Schwefels mit ein oder zwei Äquivalenten eines Oxidationsmittels um. Die Oxidationsmittel können gegebenenfalls auch im Überschuß eingesetzt werden.

Das Verfahren C zur Herstellung von Verbindungen der Formel (Ib) ist dadurch gekennzeichnet, daß man 2-Arylpyrrole der Formel (Ia) mit Verbindungen der Formel (IV), gegebenenfalls in Gegenwart von Basen und gegebenenfalls in Gegenwart von Verdünnungsmitteln umsetzt.

Als Verdünnungsmittel kommen bei Verfahren C alle inerten organischen Lösungsmittel in Frage. Hierzu gehören vorzugsweise Kohlenwasserstoffe wie Benzol, Toluol, Xylol, ferner Ether, wie Dibutylether, tert.-Butylmethylether, Tetrahydrofuran, Dioxan, außerdem polare Lösungsmittel, wie Dimethylsulfoxid, Acetonitril, Sulfolan, Dimethylformamid und N-Methyl-pyrrolidon.

Als Basen können alle üblichen Protonenakzeptoren eingesetzt werden Vorzugsweise verwendbar sind Alkalimetall- und Erdalkalimetall-oxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid oder 18-Krone-6 eingesetzt werden können. Ferner sind Alkalimetall- und Erdalkalimetallamide und -hydride, wie Natriumamid, Natriumhydrid und Calciumhydrid, und außerdem auch Alkalimetall-alkoholate, wie Natrium-methylat, Natrium-ethylat und Kalium-tert.-butylat einsetzbar.

Die Reaktionstemperaturen können bei der Durchführung in einem größeren Bereich variiert werden. Im allgemeinen arbeitet man bei Temperaturen zwischen -10°C und 200°C, vorzugsweise zwischen 0°C und 120°C.

Bei der Durchführung setzt man die Reaktionskomponenten der Formel (Ia), die deprotonierenden Basen und die Komponenten der Formel (IV) im allgemeinen in etwa äquimolaren Mengen ein. Es ist jedoch auch möglich, die eine oder andere Komponente in einem größeren Überschuß (bis zu 3 Mol) zu verwenden.

Das Verfahren C wird im allgemeinen unter Normaldruck durchgeführt, es kann aber auch bei Überdruck durchgeführt werden.

Das Verfahren D zur Herstellung von Verbindungen der Formel (Ic) ist dadurch gekennzeichnet, daß man 2-Arylpyrrole der Formel (III) mit Verbindungen der Formel (IV), gegebenenfalls in Gegenwart von Basen und gegebenenfalls in Gegenwart von Verdünnungsmitteln umsetzt.

Als Verdünnungsmittel kommen bei Verfahren D alle inerten organischen Lösungsmittel in Frage. Hierzu gehören vorzugsweise Kohlenwasserstoffe wie Benzol, Toluol, Xylol, ferner Ether, wie Dibutylether, tert.-Butylmethylether, Tetrahydrofuran, Dioxan, außerdem polare Lösungsmittel, wie Dimethylsulfoxid, Acetonitril, Sulfolan, Dimethylformamid und N-Methyl-pyrrolidon.

Als Basen können alle üblichen Protonenakzeptoren eingesetzt werden, Vorzugsweise verwendbar sind Alkalimetall- und Erdalkalimetall-oxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid oder 18-Krone-6 eingesetzt werden können, Ferner sind Alkalimetall- und Erdalkalimetallamide und -hydride, wie Natriumamid, Natriumhydrid und Calciumhydrid, und außerdem auch Alkalimetall-alkoholate, wie Natrium-methylat, Natriumethylat und Kaliumtert.-butylat einsetzbar.

Die Reaktionstemperaturen können bei der Durchführung in einem größeren Bereich variiert werden. Im allgemeinen arbeitet man bei Temperaturen zwischen -10°C und 200°C, vorzugsweise zwischen 0°C und 120°C.

Bei der Durchführung setzt man die Reaktionskomponenten der Formel (III), die deprotonierenden Basen und die Komponenten der Formel (IV) im allgemeinen in etwa äquimolaren Mengen ein. Es ist jedoch auch möglich, die eine oder andere Komponente in einem größeren Überschuß (bis zu 3 Mol) zu verwenden.

Das Verfahren wird im allgemeinen unter Normaldruck durchgeführt, es kann aber auch unter erhöhtem Druck durchgeführt werden.

Das Verfahren E zur Herstellung von Verbindungen der Formel (Id) (=III) ist dadurch gekennzeichnet, daß man 2-Arylpyrrole der Formel (V) mit Halogenierungsmitteln behandelt.

Als Verdünnungsmittel können bei Verfahren E alle üblichen Lösungsmittel eingesetzt werden. Vorzugsweise verwendbar sind Kohlenwasserstoffe wie Benzol und Hexan, halogenierte Kohlenwasserstoffe wie Chloroform und Methylenchlorid, weiterhin Ether wie Dibutylether, Tetrahydrofuran, Dioxan, außerdem organische Säuren, wie Ameisensäure und Essigsäure. Weiterhin kann auch Wasser als Verdünnungsmittel eingesetzt werden.

Als Halogenierungsmittel können alle üblichen Halogenierungsmittel eingesetzt werden. Vorzugsweise verwendet man Chlor, Brom, Natriumhypochlorit, Kaliumhypochlorit, Natriumhypobromit, Kaliumhypobromit, Sulfurylchlorid, t-Butylhypochlorit, N-Chlorsuccinimid, N-Bromsuccinimid.

Die Temperaturen können innerhalb eines größeren Bereichs variiert werden. Im allgemeinen arbeitet man bei Temperaturen von -10°C bis +120°C, vorzugsweise zwischen 0°C und 70°C.

Bei der Durchführung setzt man die Reaktionskomponenten der Formel (V) mit äquimolaren oder überschüssigen Mengen eines Halogenierungsmittels um.

Diese Umsetzung wird im allgemeinen unter Normaldruck durchgeführt. Im Falle der Verwendung von Chlor oder Brom als Halogenierungsmittel kann die Reaktion auch bei erhöhtem Druck (bis 5000 hPa) durchgeführt werden.

Die bei der Herstellung von Verbindungen der allgemeinen Formel (Ia) nach Verfahren A benötigten Ausgangsstoffe der allgemeinen Formel (II) sind neu; man erhält sie, wenn man 2-Arylpyrrole der Formel (V), 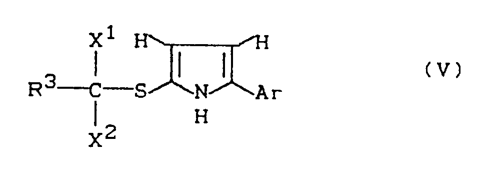
in welcher R³, X¹, X² und Ar die oben angegebene Bedeutung haben, mit Oxidationsmitteln umsetzt.

Verwendet man beispielsweise 2-(4-Chlorphenyl)-5-(difluorchlormethylthio)-pyrrol und m-Chlorperbenzoesäure als Ausgangsstoffe, so kann der Reaktionsverlauf nach diesem Verfahren durch das folgende Reaktionsschema wiedergegeben werden: 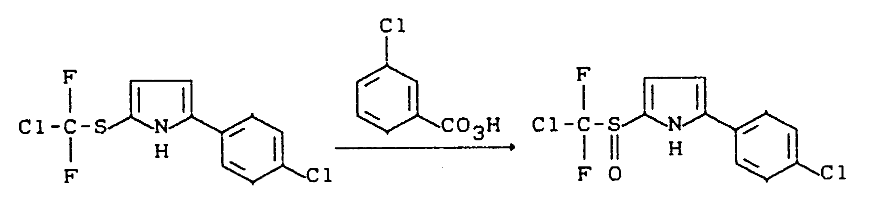
Das Verfahren zur Herstellung von Verbindungen der Formel (II) ist dadurch gekennzeichnet, daß man 2-Arylpyrrole der allgemeinen Formel (V) mit Oxidationsmitteln umsetzt.

Als Verdünnungsmittel können bei diesem Verfahren alle üblichen Lösungsmittel eingesetzt werden. Vorzugsweise verwendbar sind Kohlenwasserstoffe wie Benzol und Hexan, halogenierte Kohlenwasserstoffe wie Chloroform und Methylenchlorid, weiterhin Ether wie Dibutylether, Tetrahydrofuran, Dioxan, außerdem organische Säuren, wie Ameisensaure und Essigsäure. Weiterhin kann auch Wasser als Verdünnungsmittel eingesetzt werden.

Als Oxidationsmittel können hierbei alle üblichen Oxidationsmittel eingesetzt werden Vorzugsweise verwendet man m-Chlor-perbenzoesäure, Kaliumhydrogenperoxodisulfat (Oxone), Magnesiummonoperoxyphthalat, H₂O₂, Luftsauerstoff in Gegenwart von Katalysatoren, Kaliumpermanganat oder CrO₃.

Die Temperaturen können innerhalb eines größeren Bereichs variiert werden. Im allgemeinen arbeitet man bei Temperaturen von -30°C bis +200°C, vorzugsweise zwischen -10°C und +80°C.

Bei der Durchführung setzt man die Reaktionskomponenten der Formel (V) je nach angestrebter Oxidationsstufe des Schwefels mit ein oder zwei Equivalenten eines Oxidationsmittels um. Die Oxidationsmittel können gegebenenfalls auch im Überschuß eingesetzt werden.

Die Ausgangsstoffe der Formel (V) sind neu; man erhält sie, wenn man 2-Arylpyrrole der allgemeinen Formel (VI) 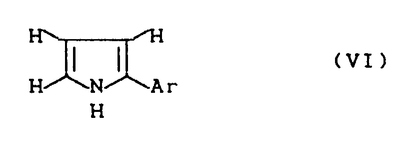
worin Ar die oben angegebene Bedeutung hat,
mit Sulfenchloriden der allgemeinen Formel (VII), 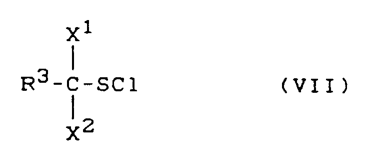
worin X¹, X² und R³ die oben angegebene Bedeutung haben, gegebenenfalls in Gegenwart von Basen und gegebenenfalls in Gegenwart von Verdünnungsmitteln umsetzt.

Verwendet man gemäß diesem Herstellungsverfahren 2-(3,4-Dichlorphenyl)-pyrrol und Trifluormethylsulfenchlorid als Ausgangsstoffe, so kann der Reaktionsverlauf durch das folgende Reaktionsschema wiedergegeben werden: 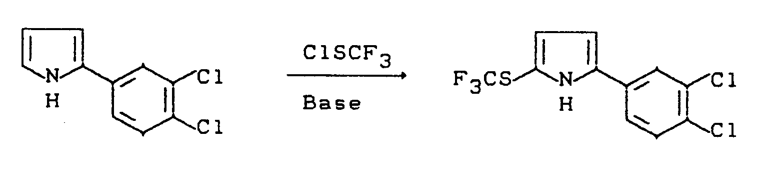
Das Verfahren zur Herstellung von Verbindungen der Formel (V) ist dadurch gekennzeichnet, daß man 2-Arylpyrrole der Formel (VI) mit Sulfenchloriden der allgemeinen Formel (VII) gegebenenfalls in Gegenwart von Basen und gegebenenfalls in Gegenwart von Verdünnungsmitteln umsetzt.

Als Verdünnungsmittel kommen bei diesem Verfahren alle inerten organischen Lösungsmittel in Frage. Hierzu gehören vorzugsweise Kohlenwasserstoffe wie Benzol, Toluol, Xylol, ferner Ether, wie Dibutylether, tert.-Butylmethylether, Tetrahydrofuran, Dioxan, außerdem polare LÖsungsmittel, wie Dimethylsulfoxid, Acetonitril, Sulfolan, Dimethylformamid und N-Methyl-pyrrolidon.

Als Basen sind z.B. Alkalimetall- und Erdalkalimetallamide und -hydride, wie Natriumamid, Natriumhydrid und Calciumhydrid, und außerdem auch Alkalimetall-alkoholate, wie Natrium-methylat, Natrium-ethylat und Kalium-tert.-butylat einsetzbar.

Die Reaktionstemperaturen können bei der Durchführung in einem größeren Bereich variiert werden. Im allgemeinen arbeitet man bei Temperaturen zwischen -70°C und +50°C, vorzugsweise zwischen -30°C und +30°C.

Bei der Durchführung setzt man die Reaktionskomponenten der Formeln (VI) und (VII) in äquimolaren Verhältnissen ein. Die Reaktionskomponenten der Formel (VII) können gegebenenfalls im Überschuß eingesetzt werden.

Die gegebenenfalls eingesetzten Basen werden im allgemeinen in äquimolaren oder leicht überschüssigen Mengen eingesetzt.

Diese Umsetzung wird im allgemeinen unter Normaldruck durchgeführt. Im Falle von gasförmigen Sulfenchloriden kann die Reaktion auch bei erhöhtem Druck (bis 5000 hPa) durchgeführt werden.

Die 2-Arylpyrrole der allgemeinen Formel (VI) sind bekannt und lassen sich nach bekannten Verfahren herstellen. (Vergl.: A. Gossauer, Die Chemie der Pyrrole, Springer-Verlag, Berlin 1974, S. 276; N. Engel, W. Steglich, Angew. Chemie 90, 719 (1978)).

Die Sulfenchloride der allgemeinen Formel (VII) sind teilweise bekannt und lassen sich nach im Prinzip bekannten Methoden herstellen. (Vergl. Houben-Weyl, Georg Thieme-Verlag Stuttgart, Bd. 9, S. 267 ff.; vgl. ferner DE-A 4 036 515).

Die erfindungsgemäßen substituierten 2-Arylpyrrole (I) eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.

Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.

Aus der Ordnung der Isoptera z.B. Reticulitermes spp.. Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.

Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen substituierten 2-Arylpyrrole (I) wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge, Flöhe.

Sie sind gegen normalsensible und resistente Arten und Stämme, sowie gegen alle parasitierenden und nicht parasitierenden Entwicklungsstadien der Ektoparasiten wirksam.

Die erfindungsgemäßen substituierten 2-Arylpyrrole (I) zeichnen sich durch eine hohe insektizide Wirksamkeit aus. Sie lassen sich mit gutem Erfolg gegen pflanzenschädigende Insekten, wie beispielsweise gegen die Larven der Meerrettichblattkäfer (Phaedon cochleariae), gegen die grüne Pfirsichblattlaus (Myzus persicae) oder gegen die schwarze Bohnenblattlaus (Aphis fabae) einsetzen, Dabei zeigen die erfindungsgemäßen Wirkstoffe nicht nur protektive sondern auch blattsystemische und wurzelsystemische Eigenschaften.

Daneben eignen sich die erfindungsgemäßen substituierten 2-Arylpyrrole (I) auch zur Bekämpfung von Bodeninsekten und lassen sich beispielsweise zur Bekämpfung der Maden der Zwiebelfliege (Phorbia antiqua) im Boden einsetzen.

Außerdem besitzen die erfindungsgemäßen substituierten 2-Arylpyrrole (I) eine hohe Wirkung gegen Hygiene- und Vorratsschädlinge und lassen sich beispielsweise zur Bekämpfung der deutschen Schabe (Blattella germanica) einsetzen.

Schließlich eignen sich die neuen Wirkstoffe der Formel (I) auch zur Bekämpfung planzenpathogener Pilze; so zeigen die Wirkstoffe eine Wirkung gegen Pyricularia oryzae, z.B. am Reis.

Die erfindungsgemäßen substituierten 2-Arylpyrrole (I) können in die üblichen Formulierungen übergeführt werden, wie Lösungen, Emulsionen, Suspensionen, Pulver, Schäume, Pasten, Granulate, Aerosole, Wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der erfindungsgemäßen substituierten 2-Arylpyrrole (I) mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Träge stoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen substituierten 2-Arylpyrrole (I) können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stofee u.a.

Die erfindungsgemäßen substituierten 2-Arylpyrrole (I) können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäß substituierten 2-Arylpyrrole (I) eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäßen substituierten 2-Arylpyrrole (I) geschieht auf diesem Gebiet in bekannter Weise wie durch orale Anwendung in Form von beispielsweise Tabletten, Kapseln, Tränken, Granulaten, durch dermale bzw. äußerliche Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion sowie ferner durch das "feed-through"-Verfahren, Daneben ist auch eine Anwendung als Formkörper (Halsband, Ohrmarke) möglich.

Die nachfolgenden Beispiele dienen zur weiteren Erläuterung der Erfindung.

### Herstellungsbeispiele

### Beispiel 1 (Verfahren A)

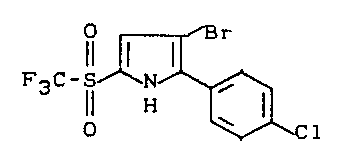

1,9 g (6,1 mmol) 2-(4-Chlorphenyl)-5-(trifluormethylsulfonyl)-pyrrol werden in 50 ml Chloroform gelöst, mit 2,4 ml (47 mmol) Brom versetzt und 10 h bei Raumtemperatur gerührt. Anschließend dampft man die Lösung im Rotationsverdampfer ein und codestilliert zweimal mit Toluol.
- Ausbeute:: 2,3 g (= 97 % d. Th.) 2-(4-Chlorphenyl)-3-brom-5-(trifluormethylsulfonyl)-pyrrol;
Physikalische Daten siehe Tabelle 1.

### Beispiel 2 (Verfahren B)

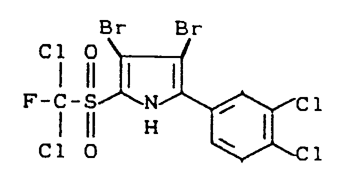

2,86 g (5,7 mmol) 2-(3,4-Dichlorphenyl)-3,4-dibrom-5-(dichlorfluormethylthio)-pyrrol werden in 35 ml Chloroform gelöst und auf -25°C gekühlt. Innerhalb von 8 Minuten wird eine Lösung von 3,6 g m-Chlorperbenzoesäure (55%ig) (11,5 mmol) in 50 ml Chloroform zugetropft. Man rührt 5 h bei Raumtemperatur nach. Anschließend wird die Reaktionslösung zweimal mit je 50 ml Na₂SO₄-Lösung und zweimal mit je 50 ml 10%iger Na₂CO₃-Lösung gewaschen. Die organische Phase wird über Na₂SO₄ getrocknet und im Rotationsverdampfer eingedampft.
- Ausbeute:: 2,5 g (= 87 % d. Th.) 2-(3,4-Dichlorphenyl)-3,4-dibrom-5-(dichlorfluormethylsulfonyl)-pyrrol;
Physikalische Daten siehe Tabelle 1.

### Beispiel 3 (Verfahren C)

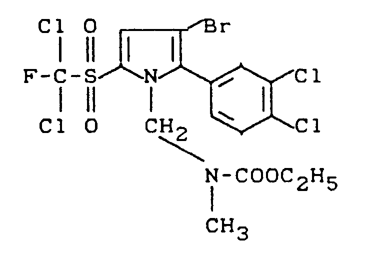

3 g (6,6 mmol) 2-(3,4-Dichlorphenyl)-3-brom-5-(dichlorfluormethylsulfonyl)-pyrrol werden in 80 ml trockenem Tetrahydrofuran gelöst und mit 0,8 g (6,0 mmol) Kalium-tert.-butylat versetzt. Hierzu tropft man eine Lösung von 1,1 g (7,2 mmol) N-Methyl-N-Chlormethyl-carbaminsäureethylester, gelöst in Tetrahydrofuran. Man rührt 17 h bei Raumtemperatur, gibt dann auf Wasser und extrahiert mit Dichlormethan. Nach Trocknen über Na₂SO₄ wird eingedampft und der Rückstand an Kieselgel (Petrolether/CH₂Cl₂ = 1:1) chromatographiert.
- Ausbeute:: 1,4 g (= 37 % d.Th.) 1-(N-Methyl-ethoxycarbonylamino-methyl)-2-(3,4-dichlorphenyl)-3-brom-5-(dichlorfluormethylsulfonyl)-pyrrol;
Physikalische Daten siehe Tabelle 1.

### Beispiel 4 (Verfahren D)

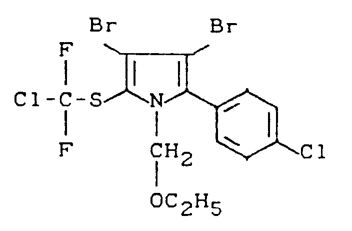

3,0 g (6,64 mmol ) 2-(4-Chlorphenyl)-3,4-dihrom-5-(difluorchlormethylthio)-pyrrol werden in 55 ml trockenem Tetrahydrofuran gelöst und mit 0,78 g (6,9 mmol) Kaliumtert.-butylat versetzt. Hierzu tropft man 0,69 g (7,3 mmol) Chlormethyl-ethylether, gelöst in ^{~}20 ml Tetrahydrofuran, und rührt 19 h bei Raumtemperatur. Anschließend gibt man auf Wasser, extrahiert mit Dichlormethan und wäscht die organische Phase zweimal mit Wasser. Man trocknet über MgSO₄ und dampft ein.
- Ausbeute:: 2,96 g (= 87 % d. Th.) 1-(Ethoxymethyl)-2-(4-chlorphenyl)-3,4-dibrom-5-(difluorchlormethyl)-pyrrol;
Physikalische Daten siehe Tabelle 1.

Analog zu den Beispielen 1 bis 4 und entsprechend den obigen allgemeinen Angaben zu den erfindungsgemäßen Herstellungsverfahren A bis E können auch die in der nachstehenden Tabelle 1 aufgeführten Verbindungen der Formel (I) hergestellt werden:

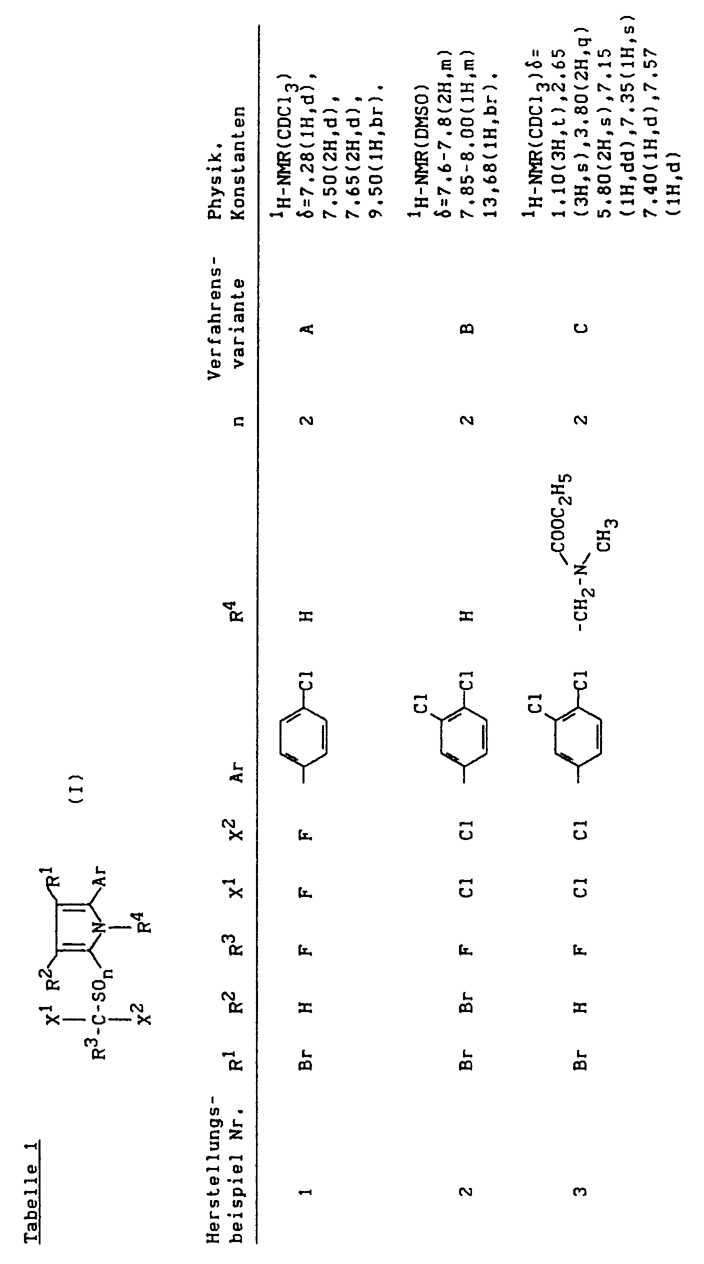

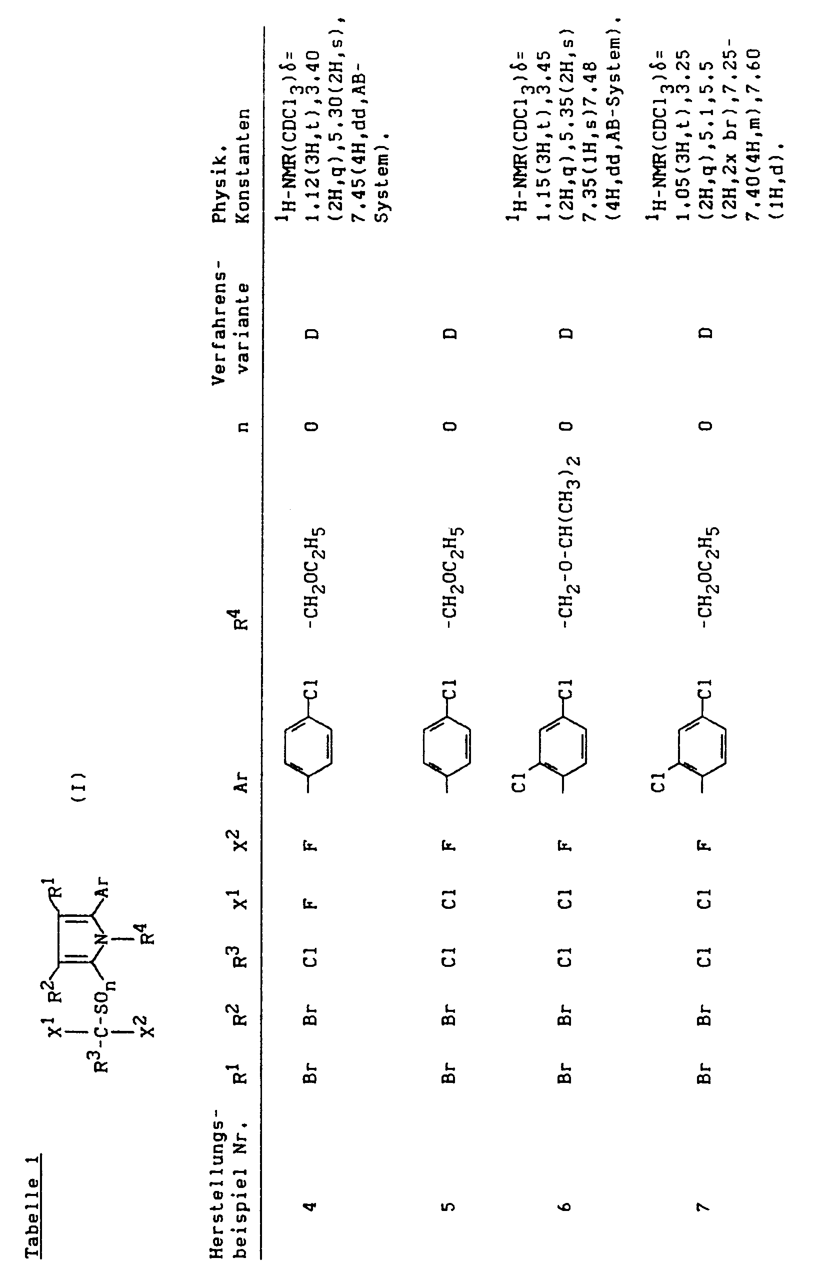

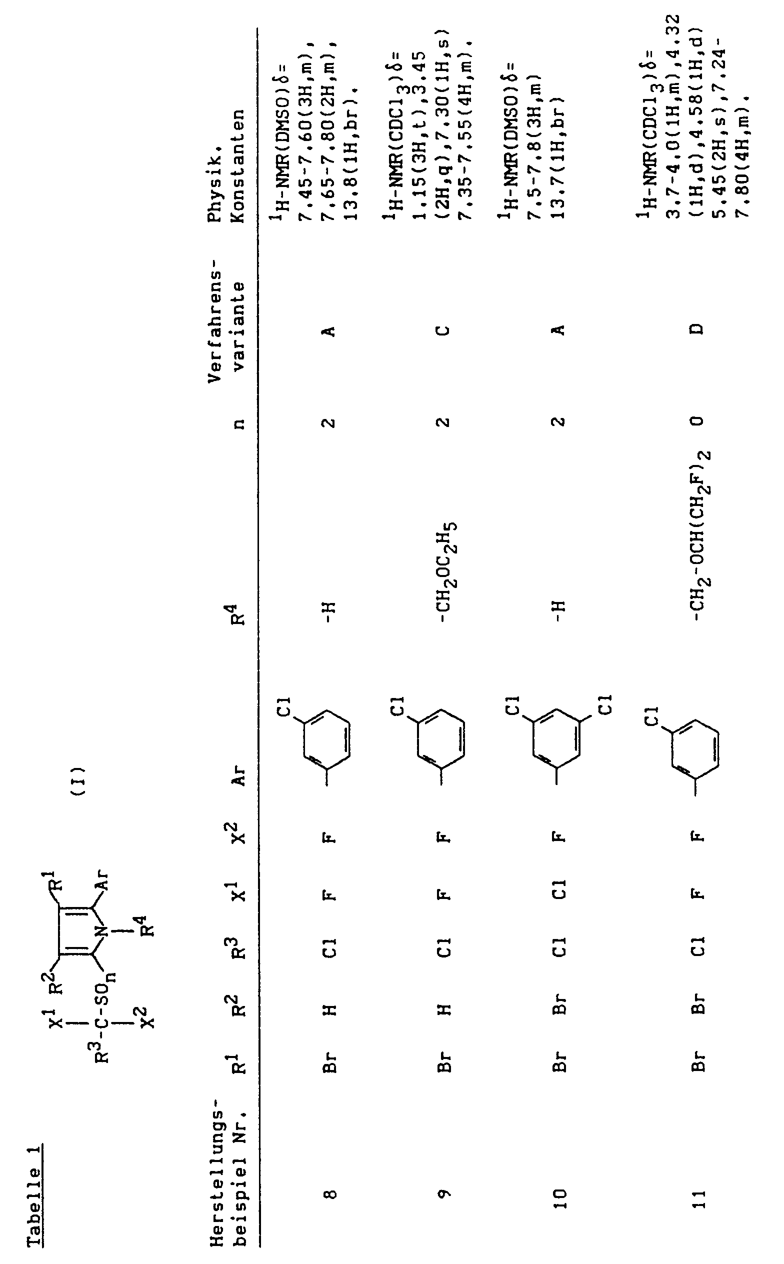

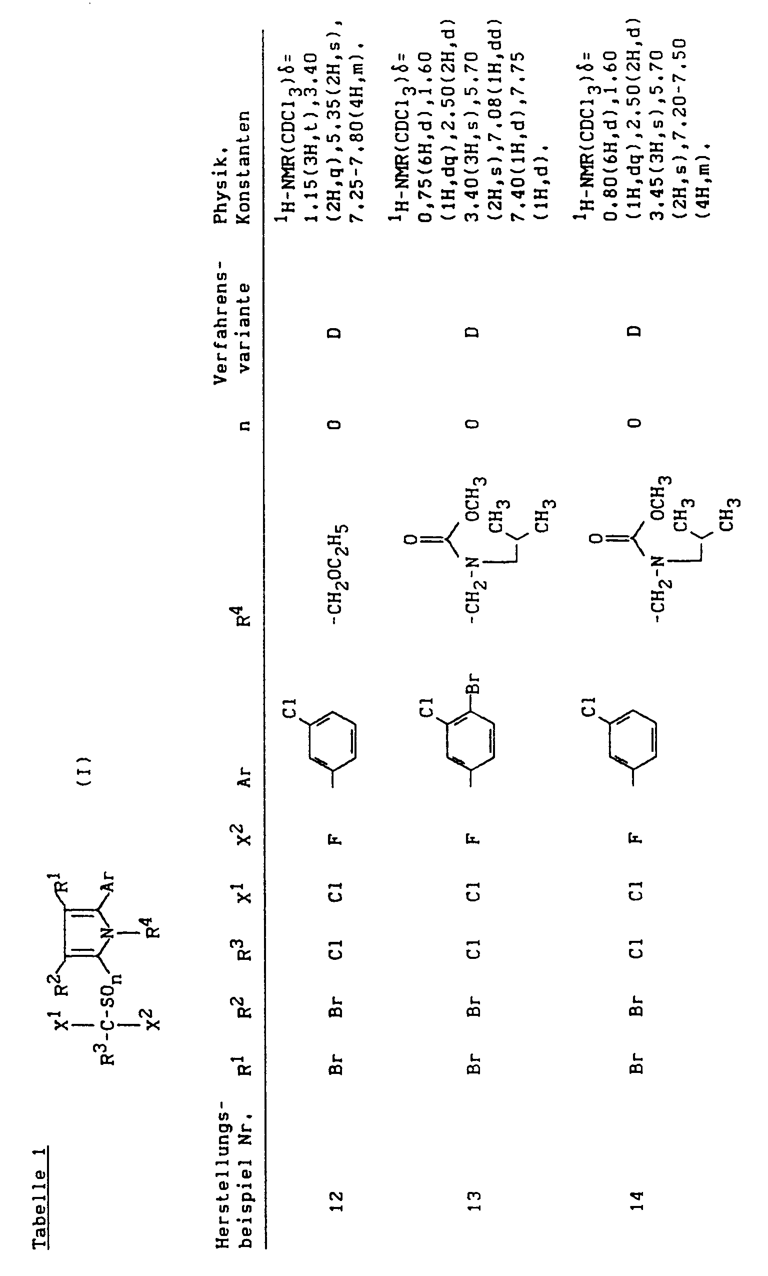

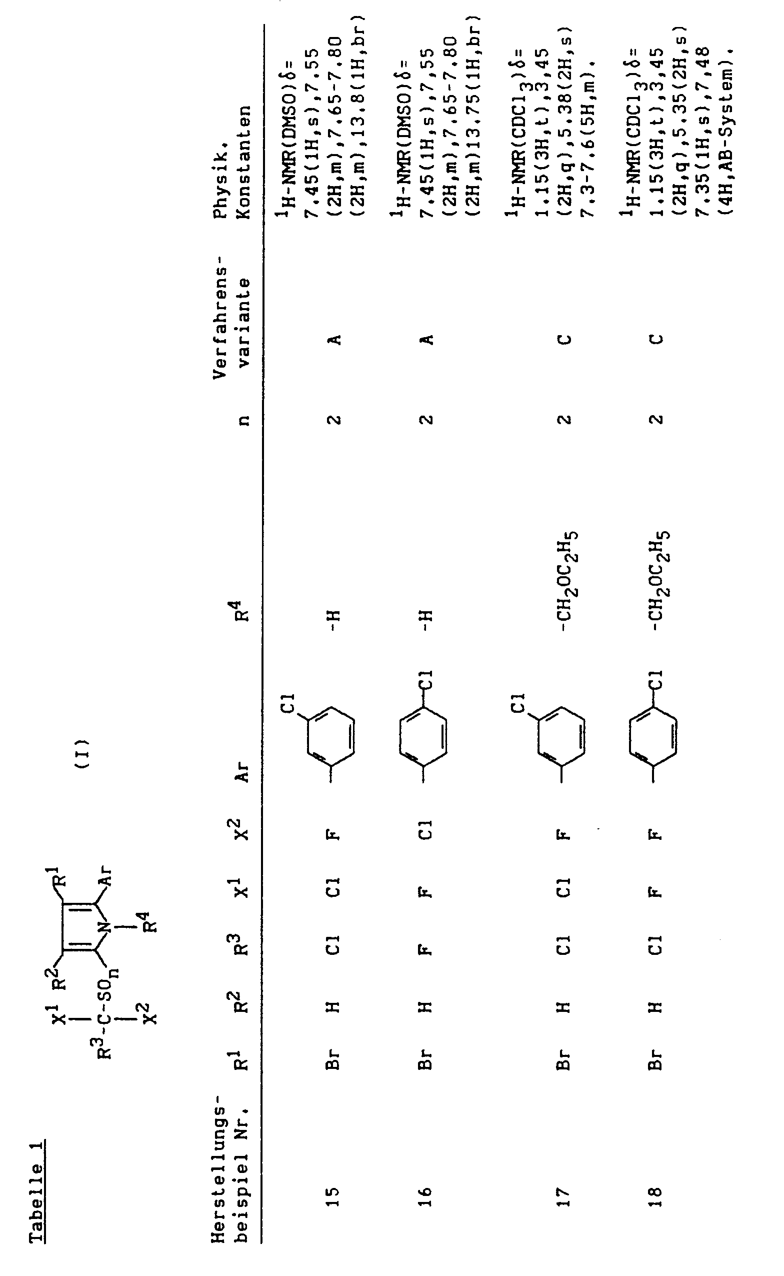

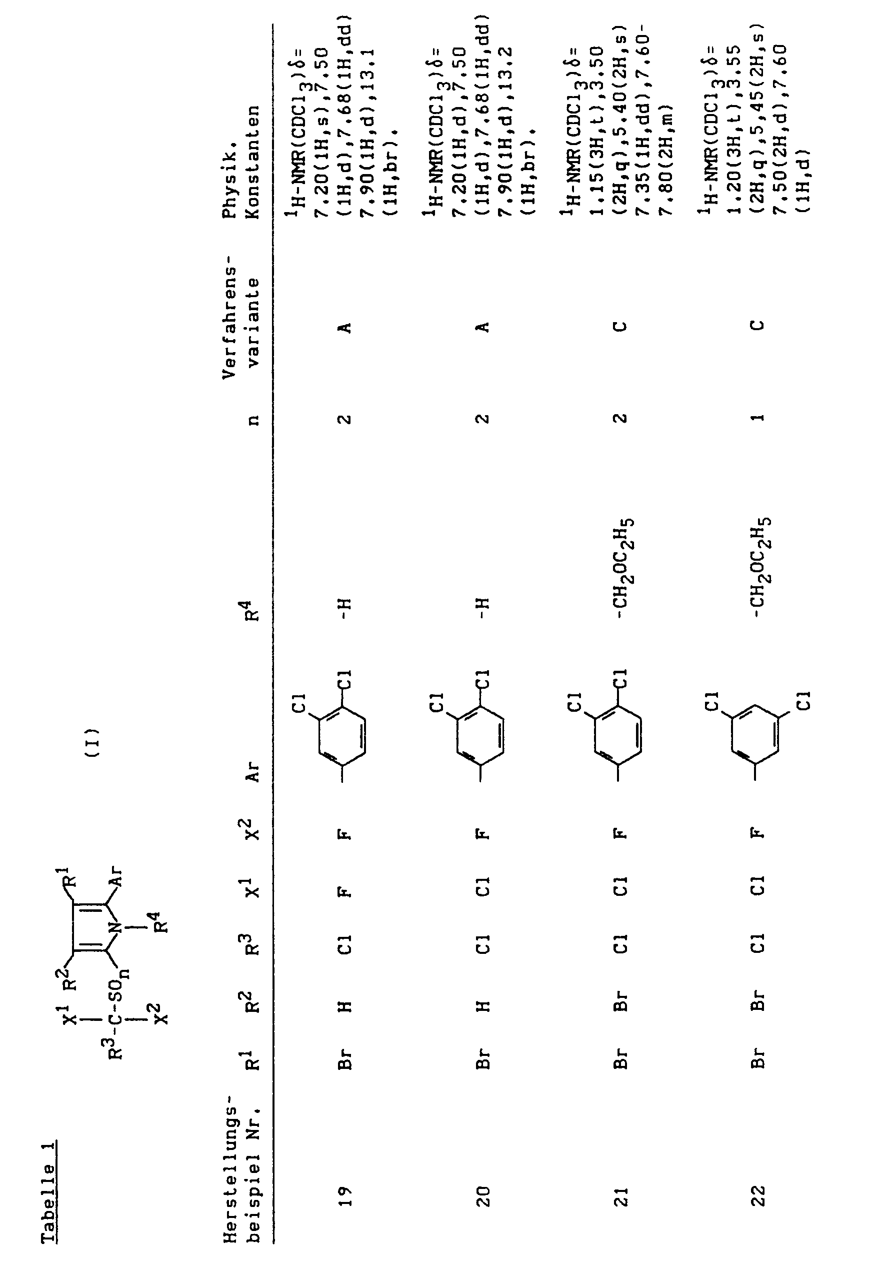

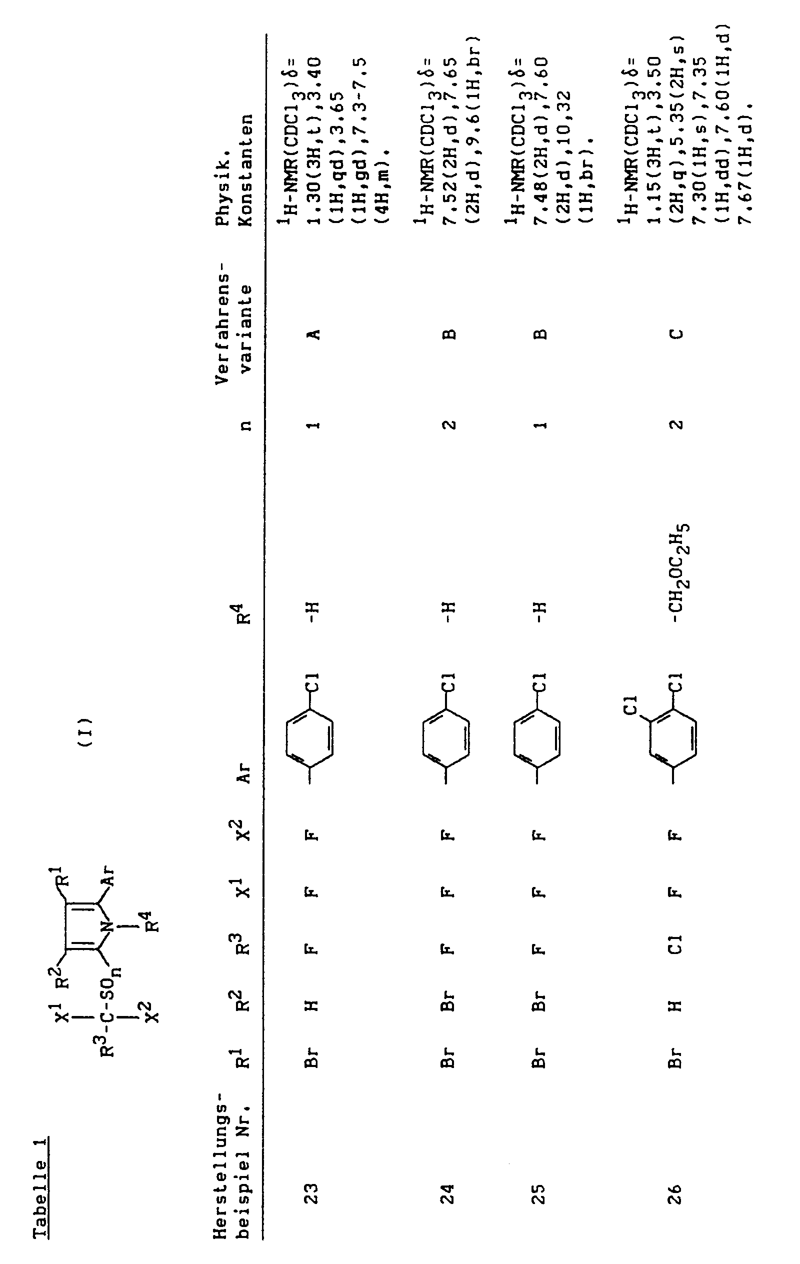

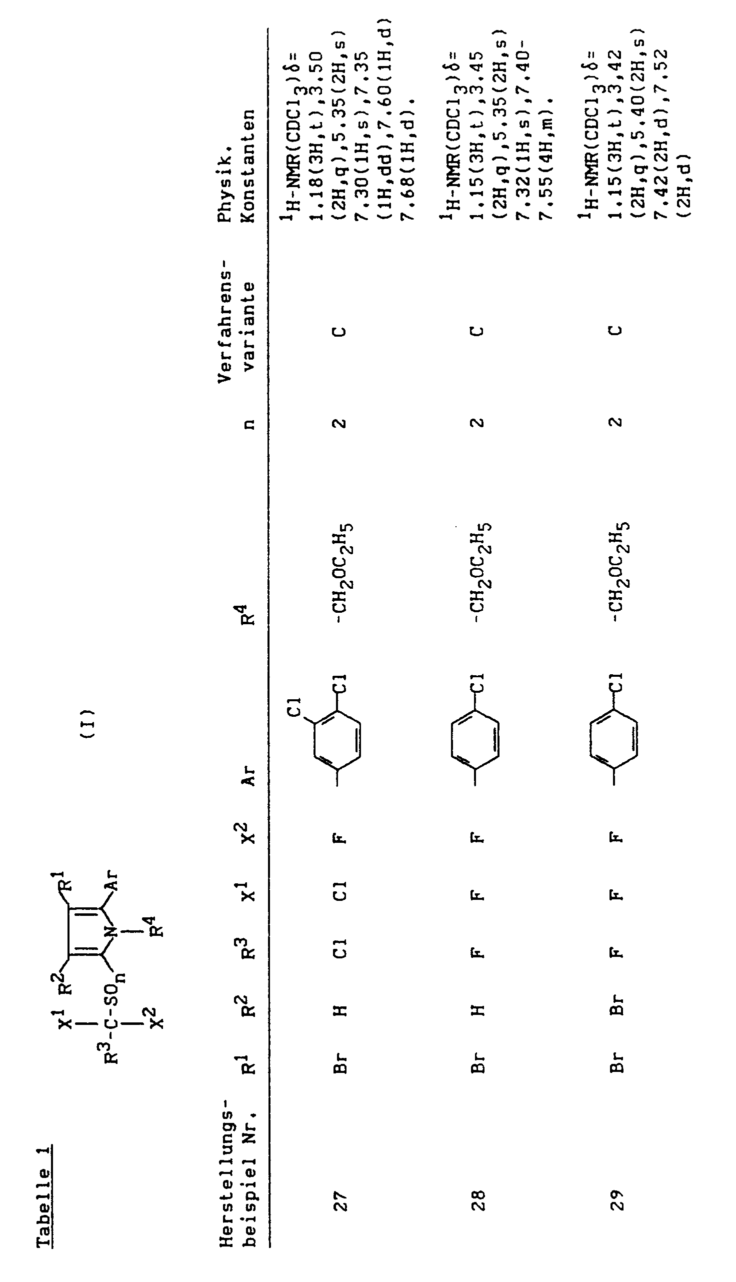

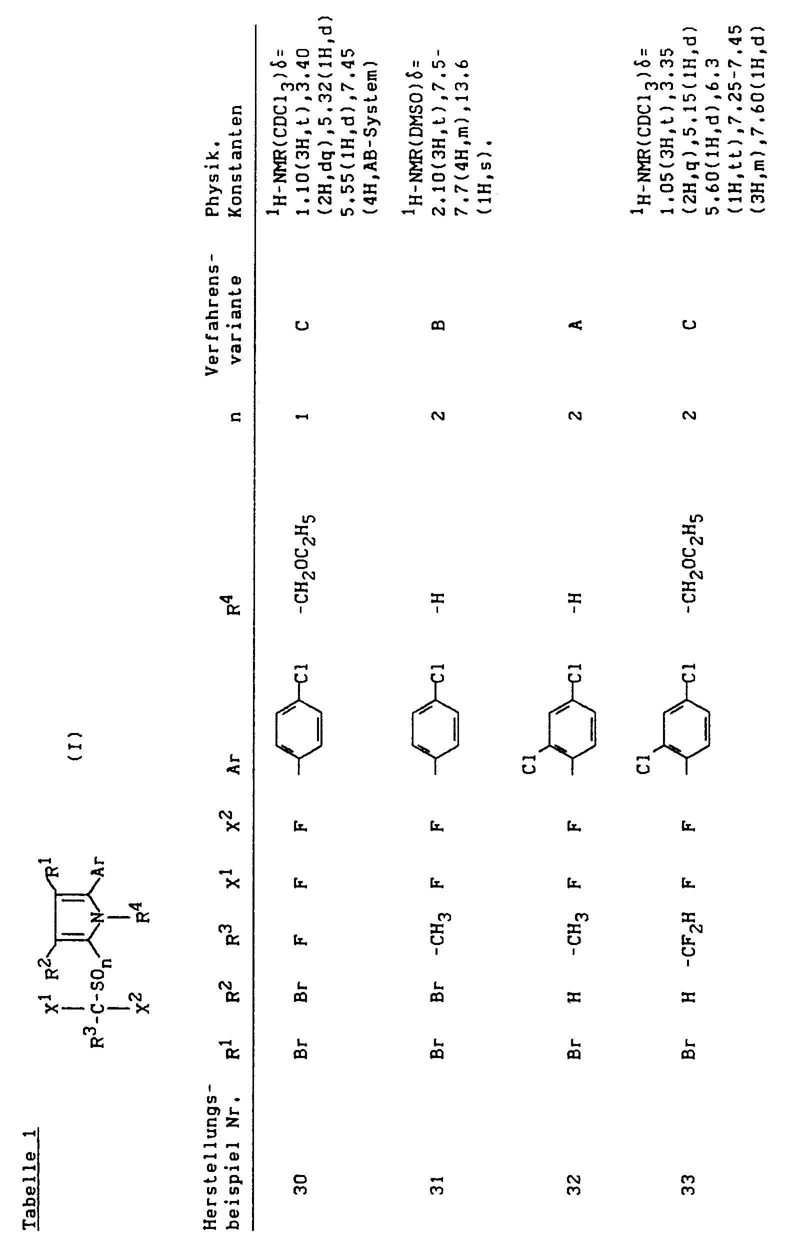

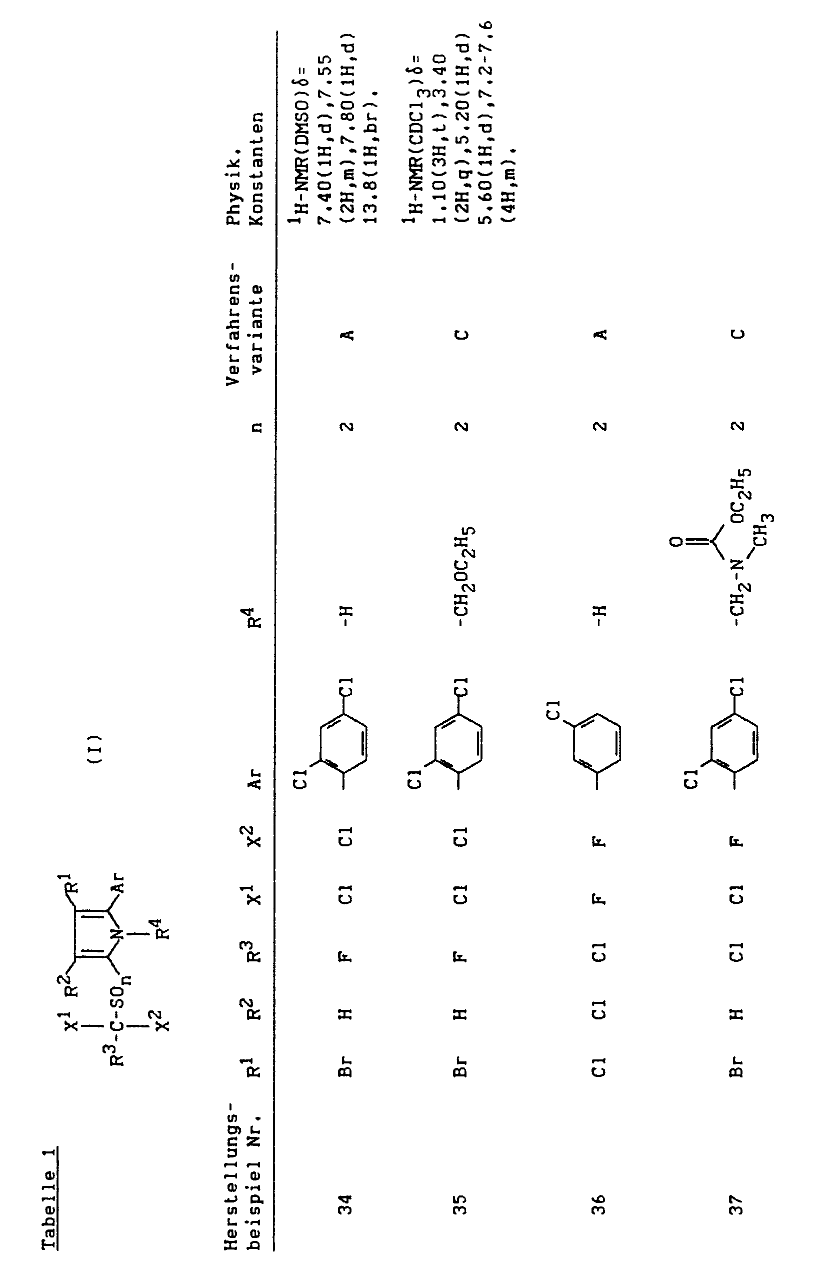

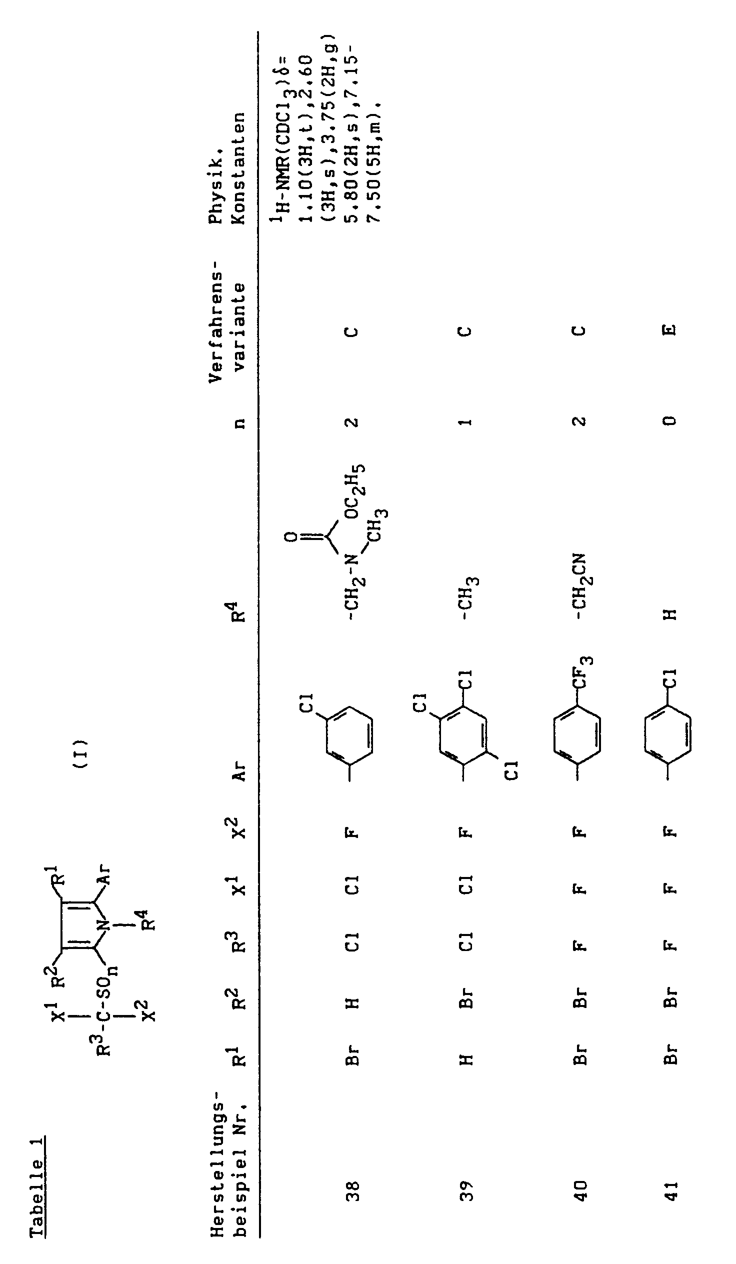

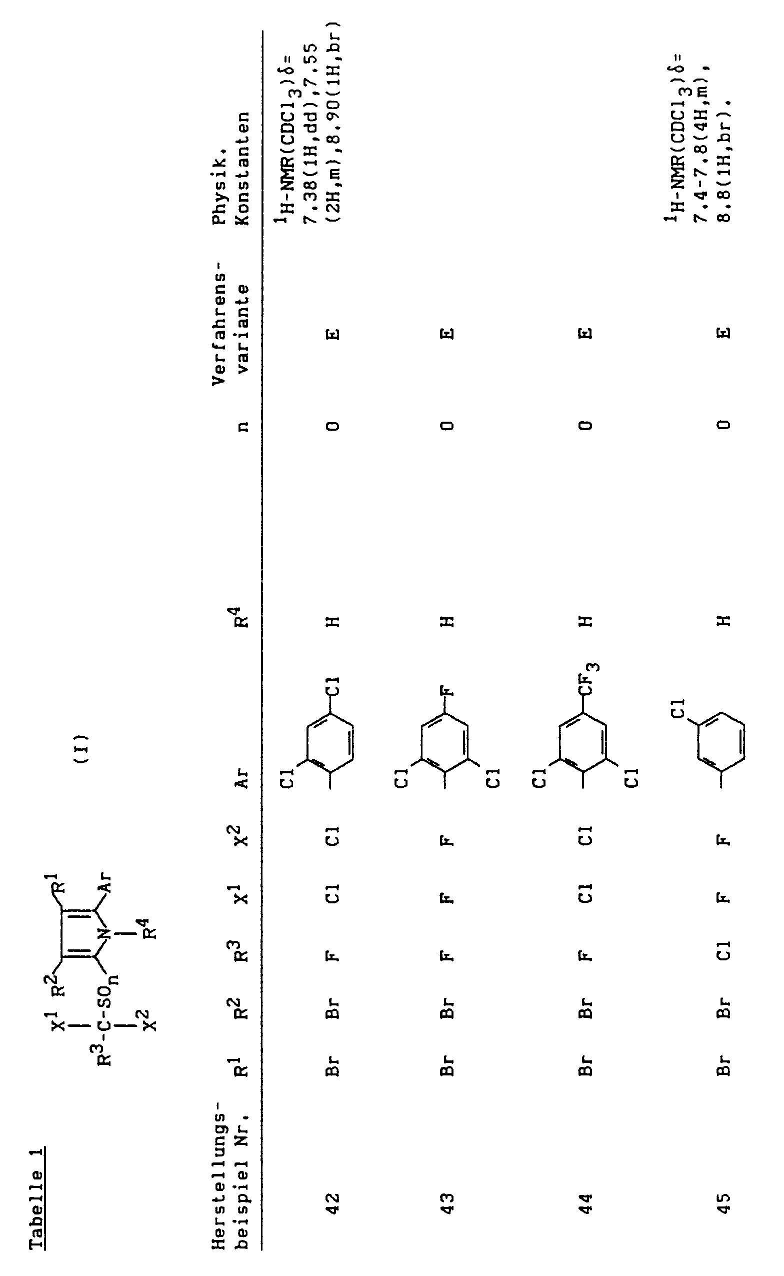

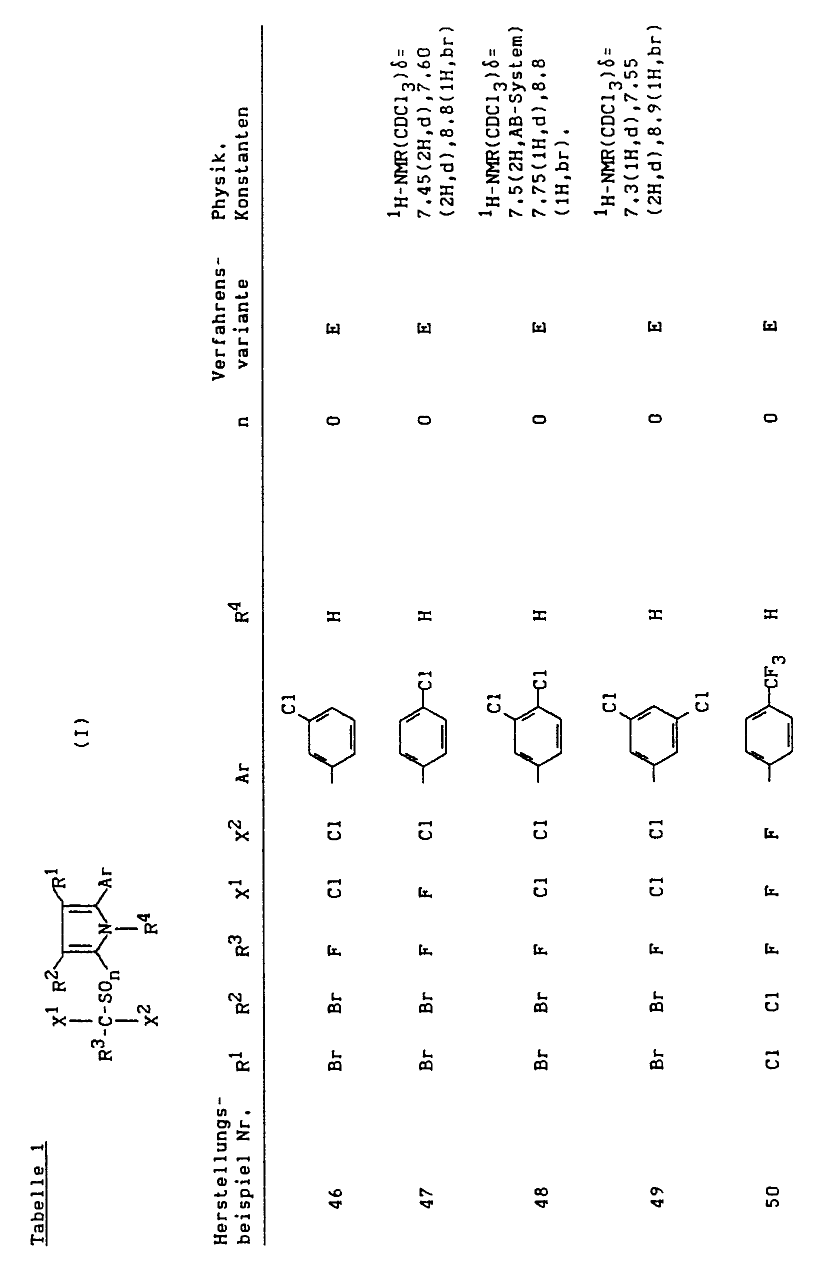

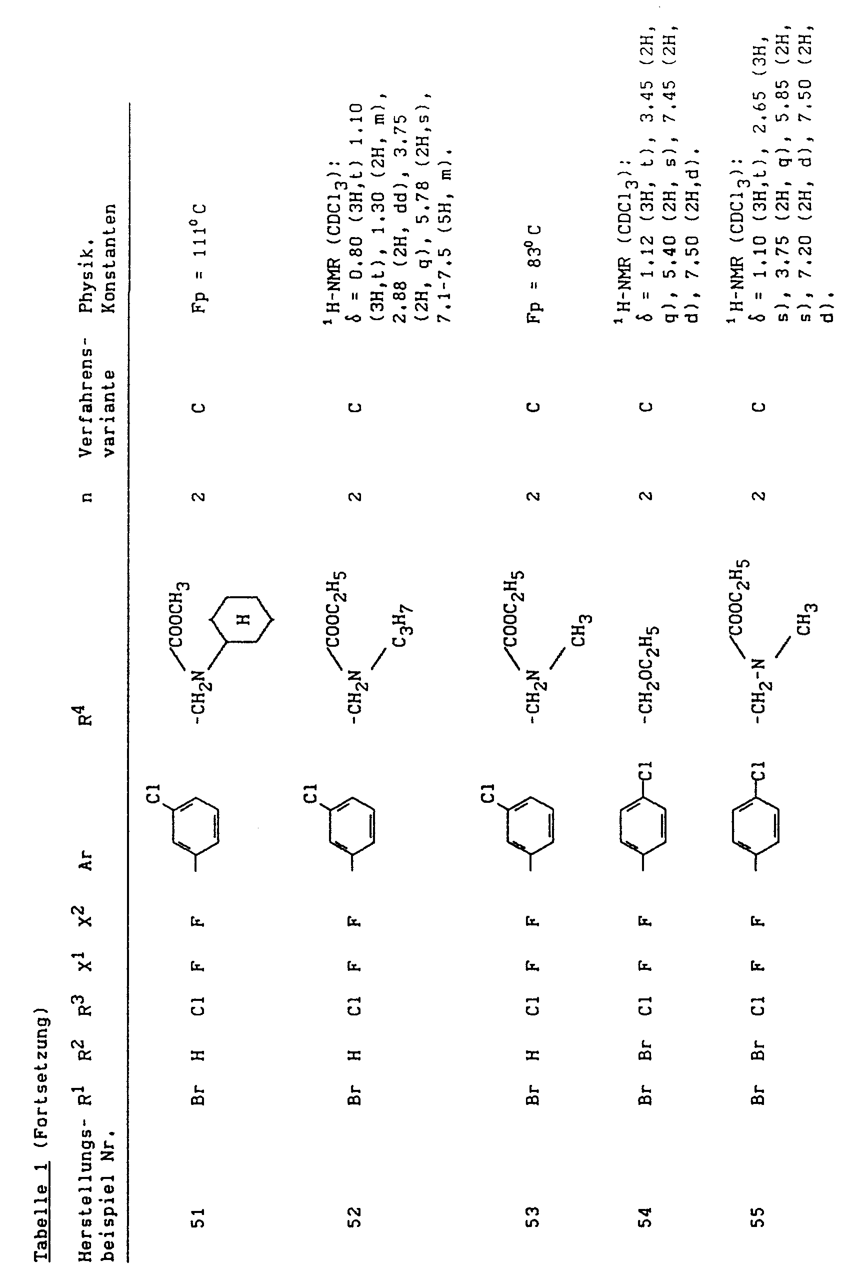

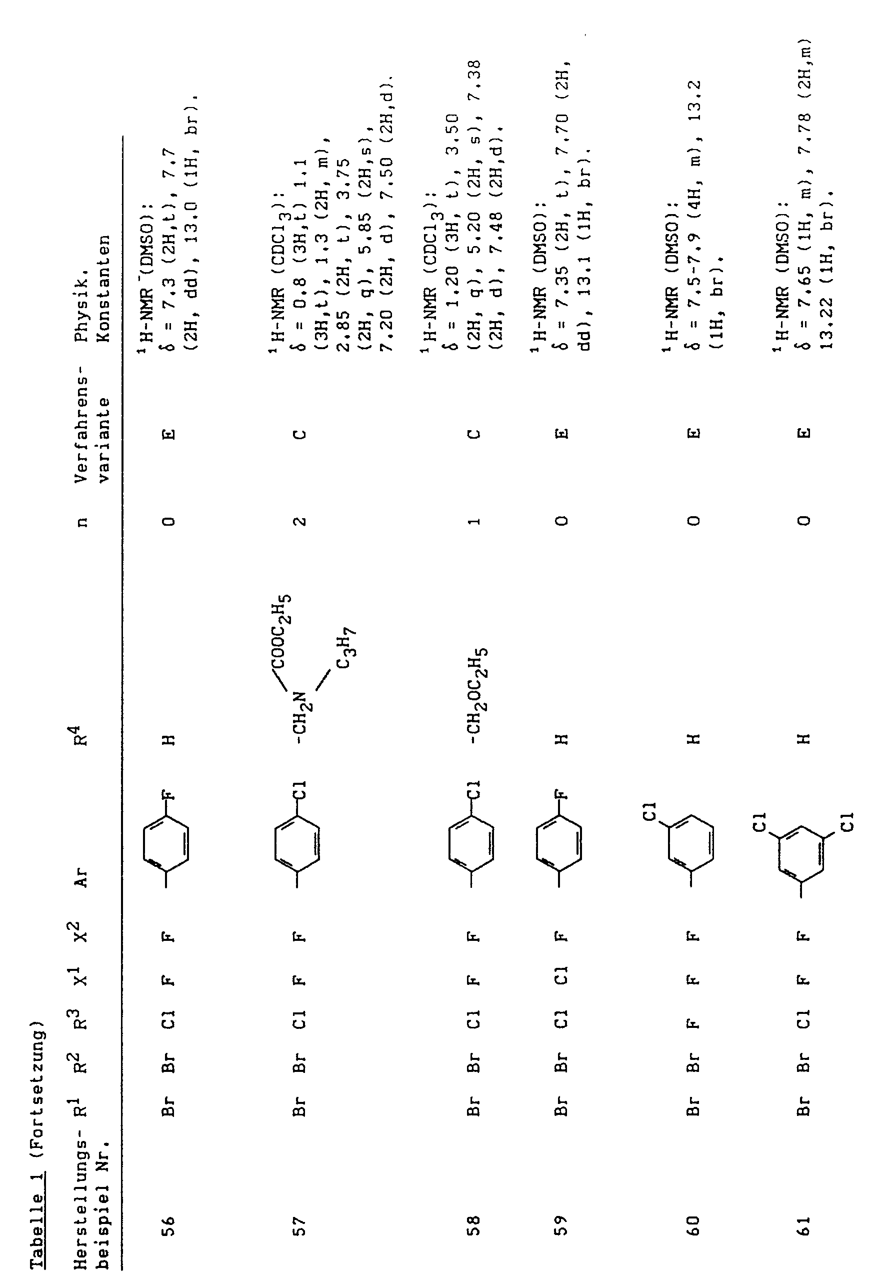

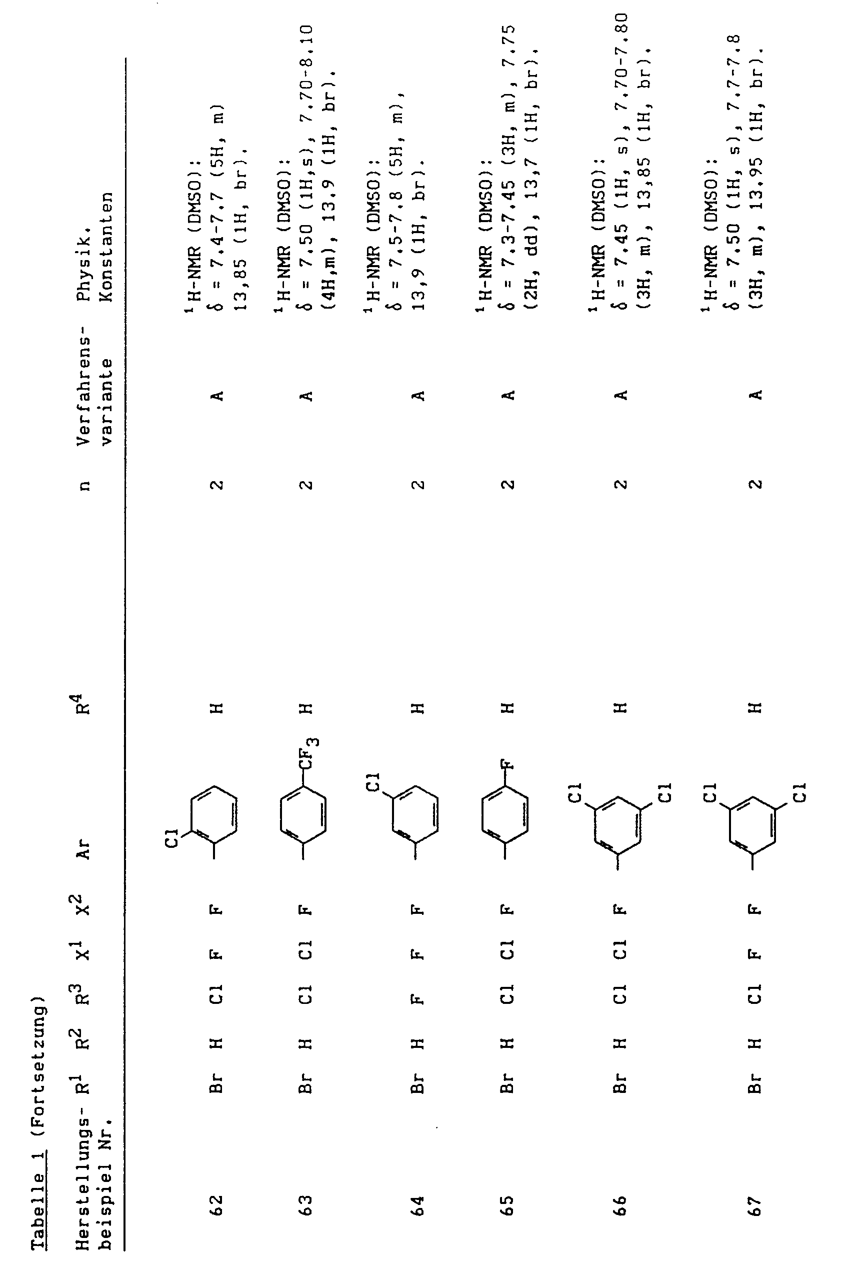

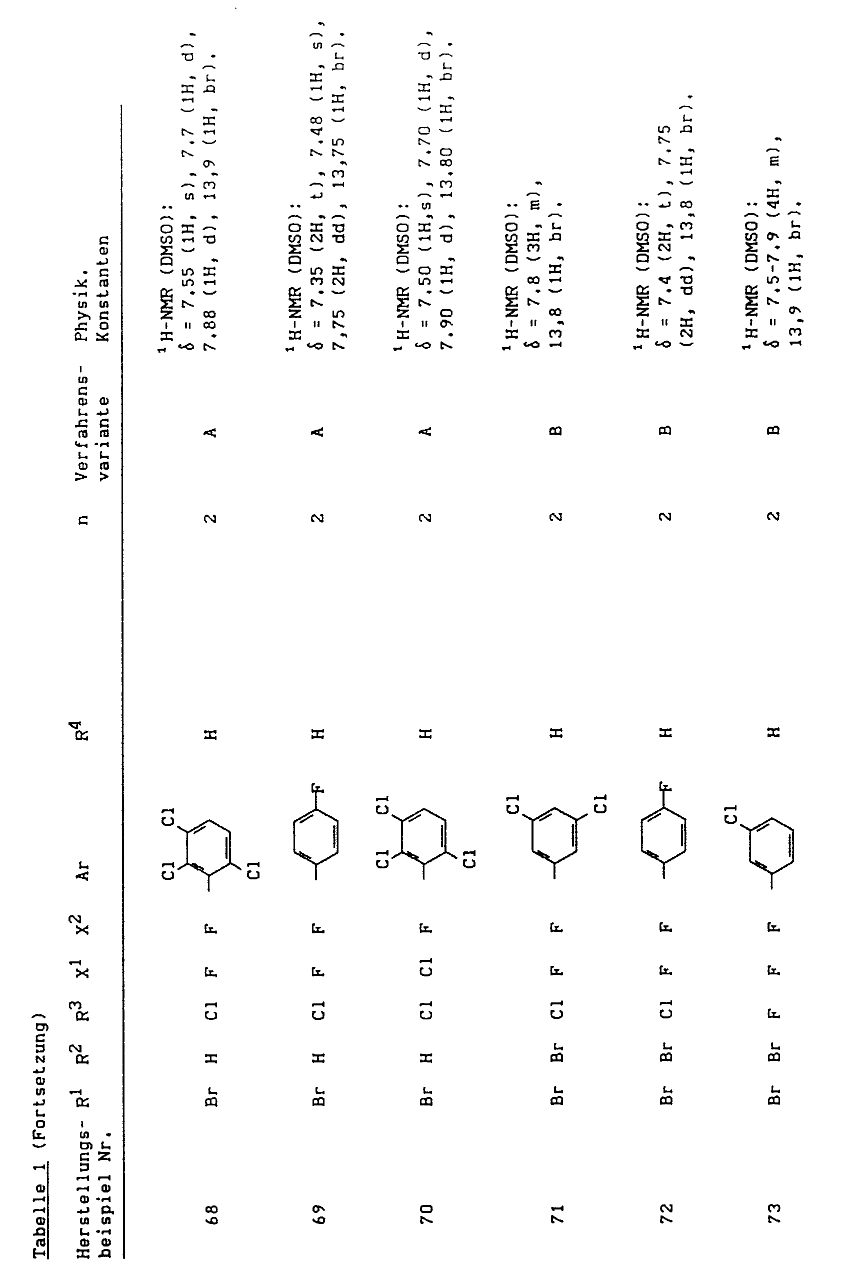

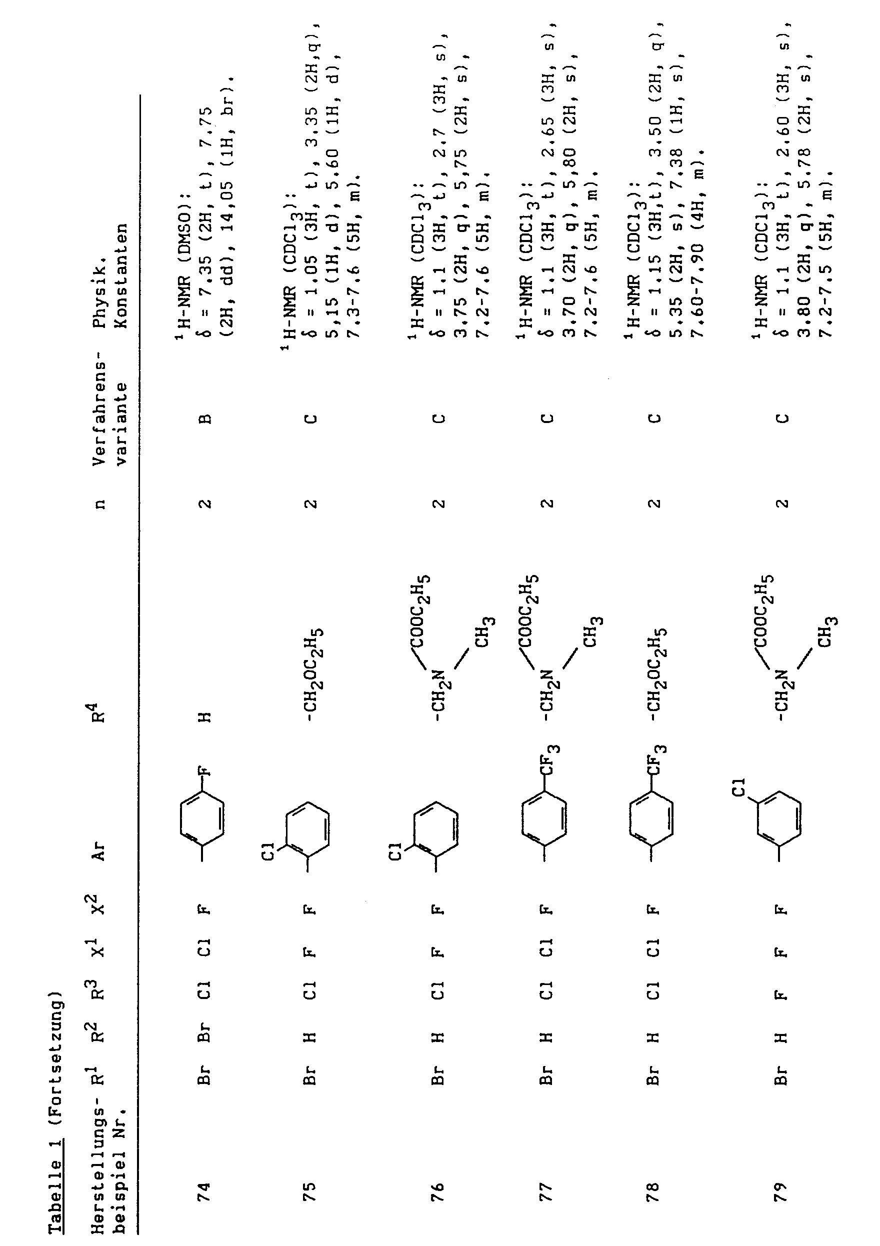

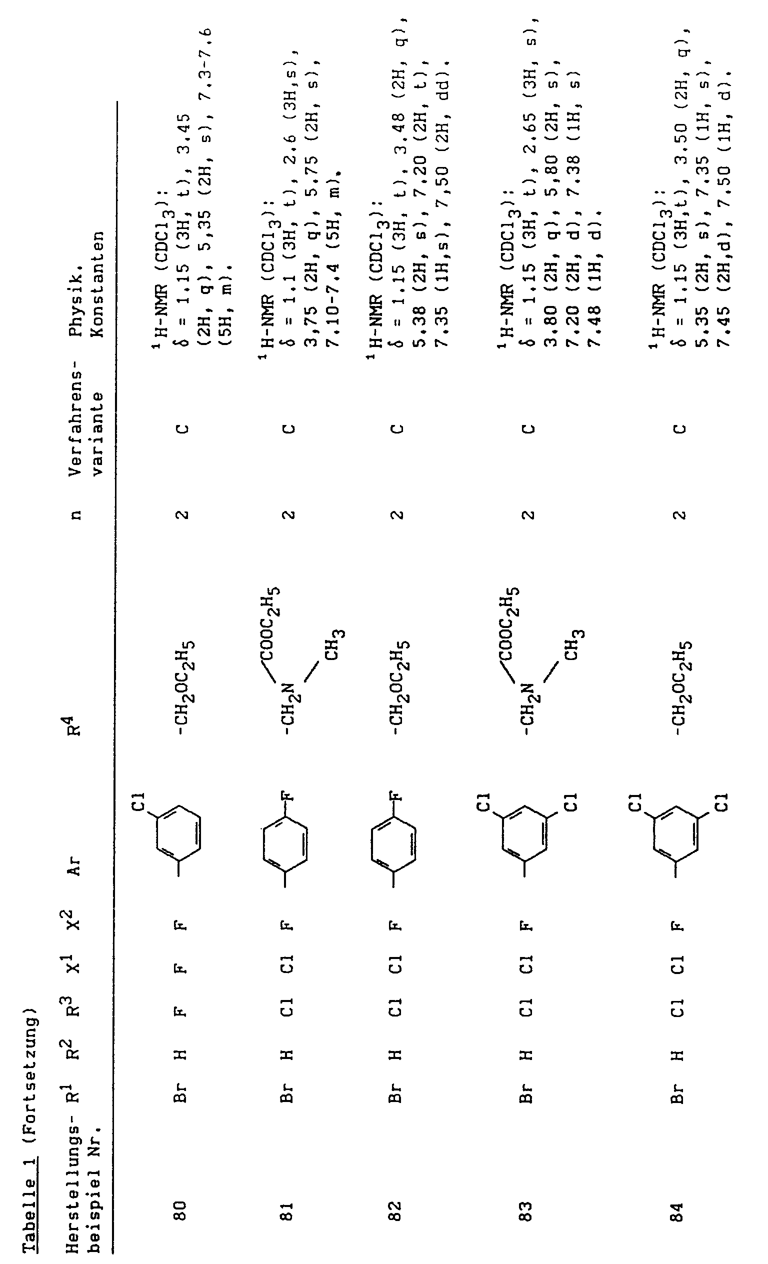

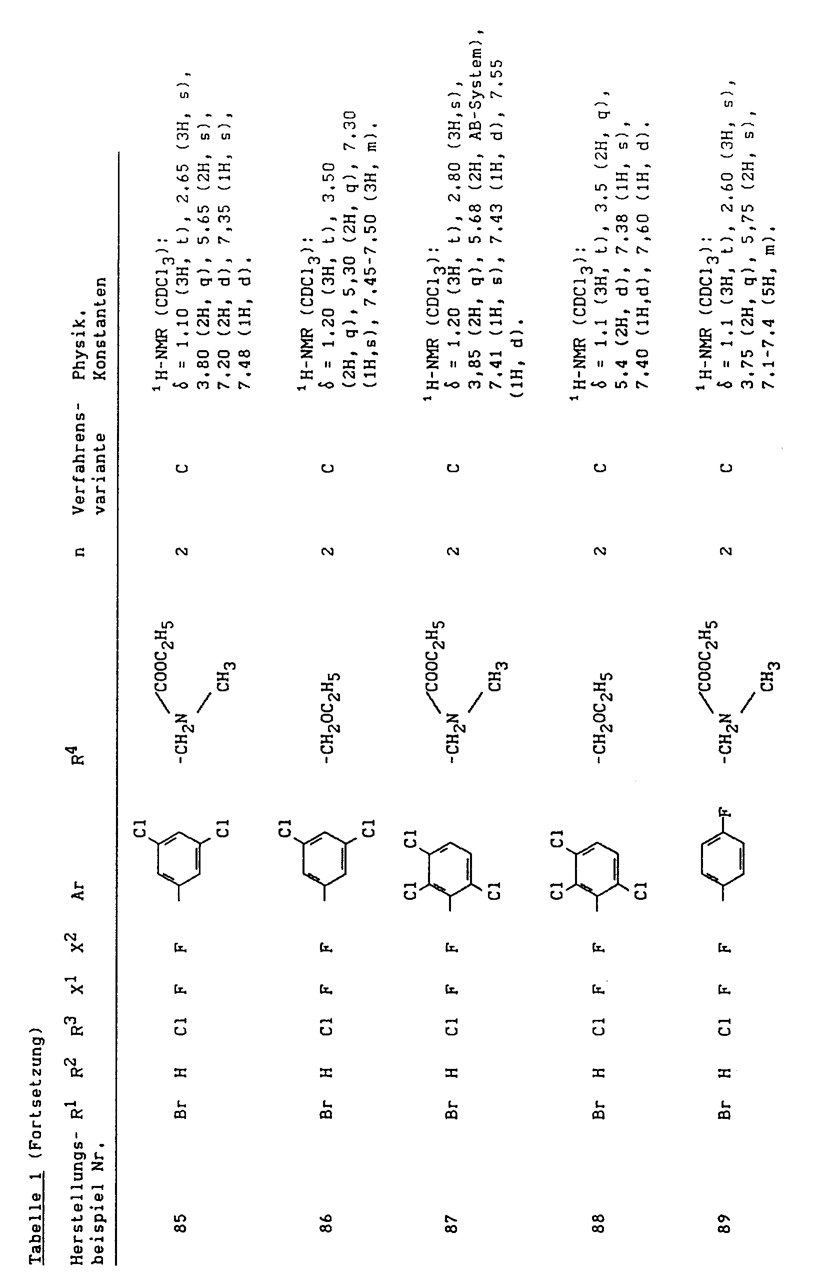

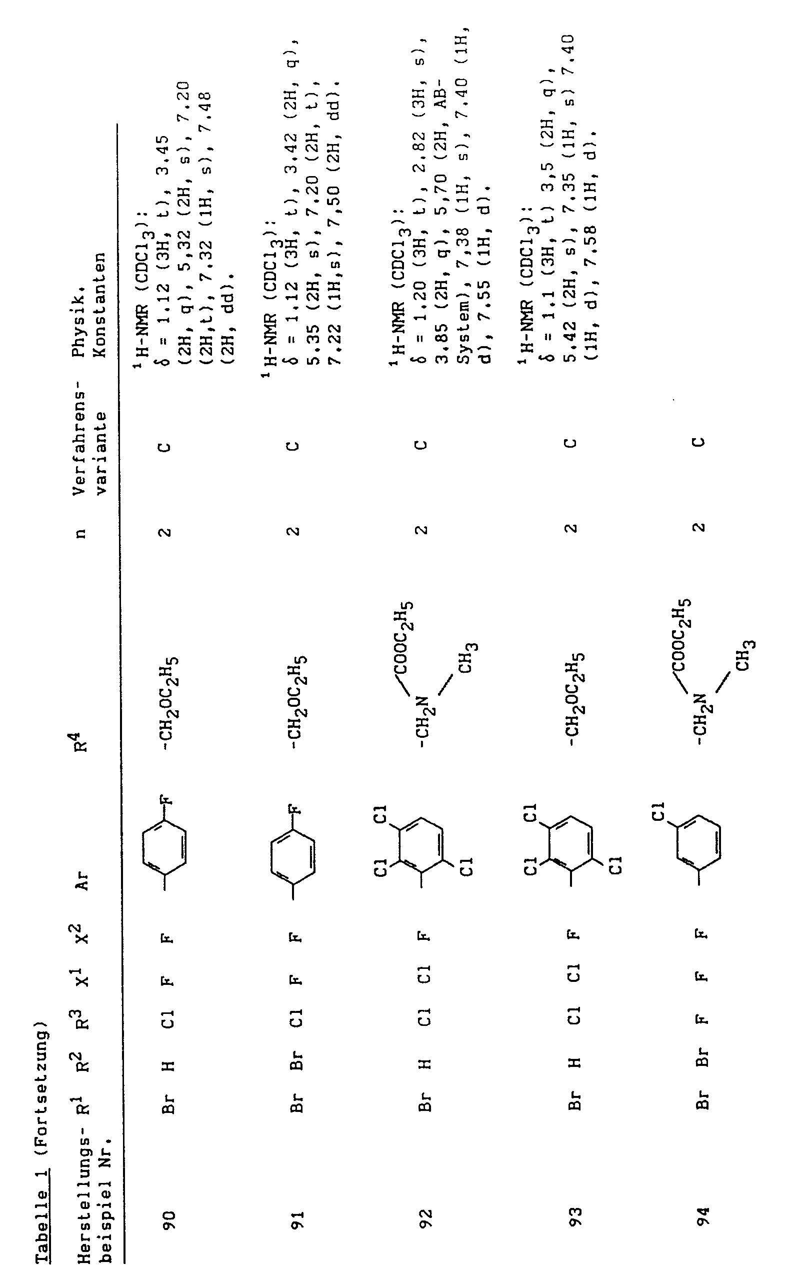

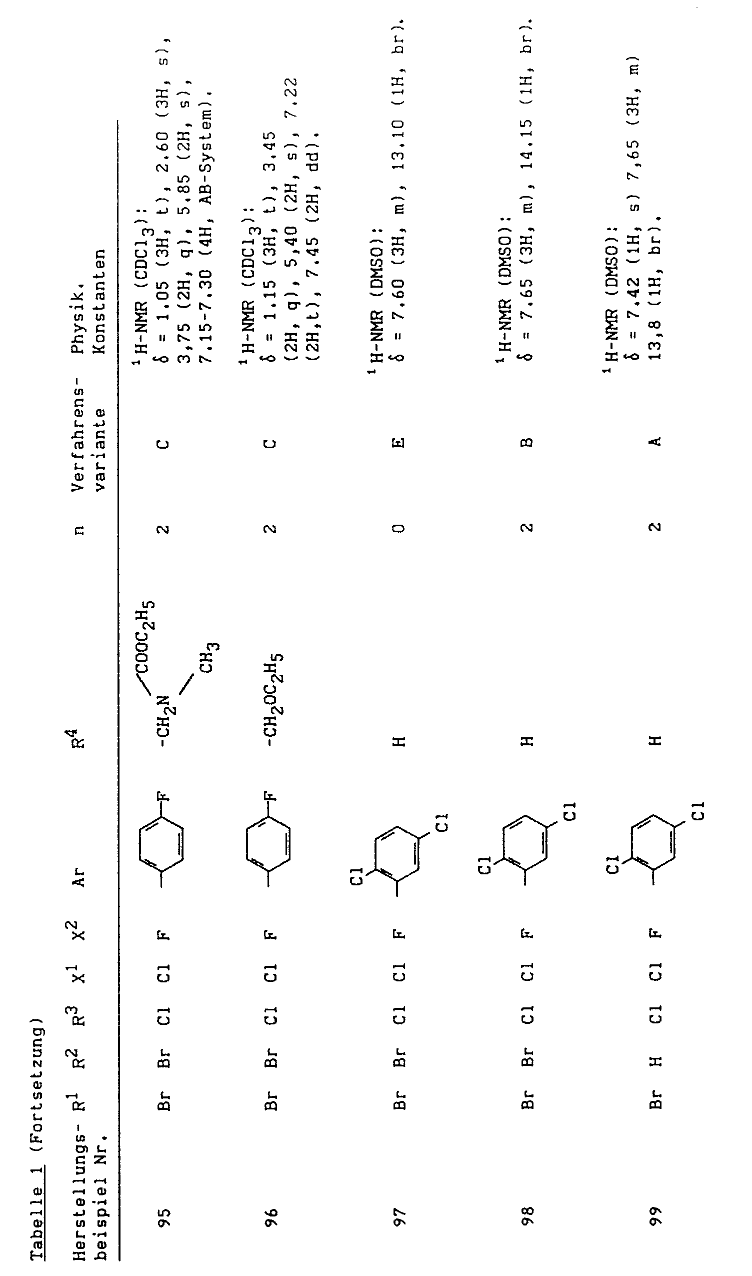

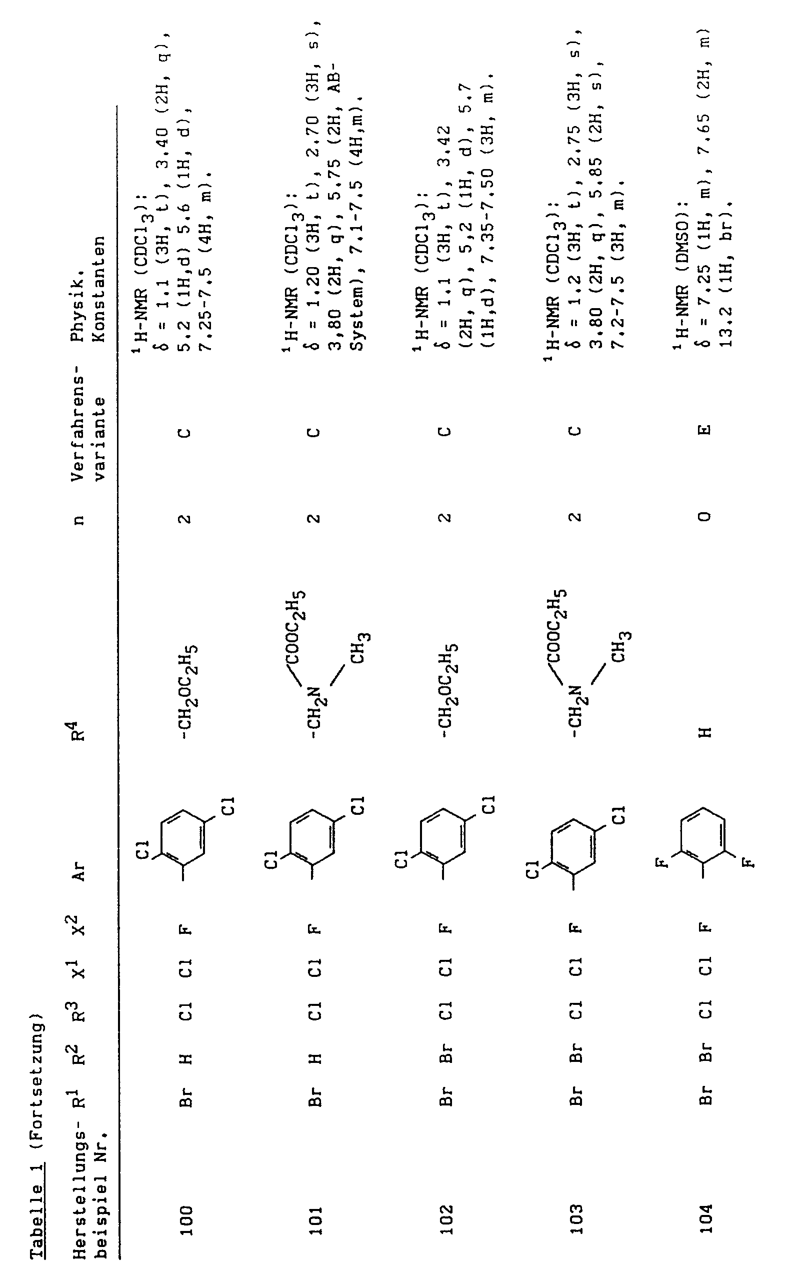

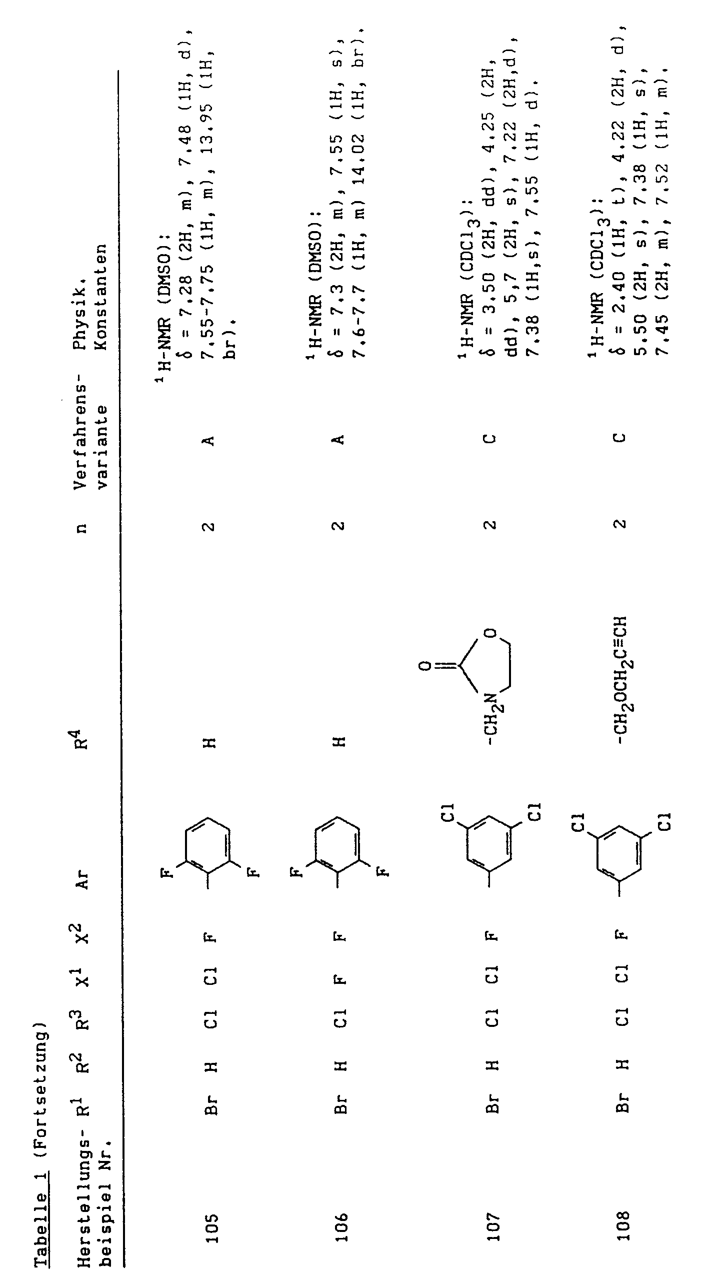

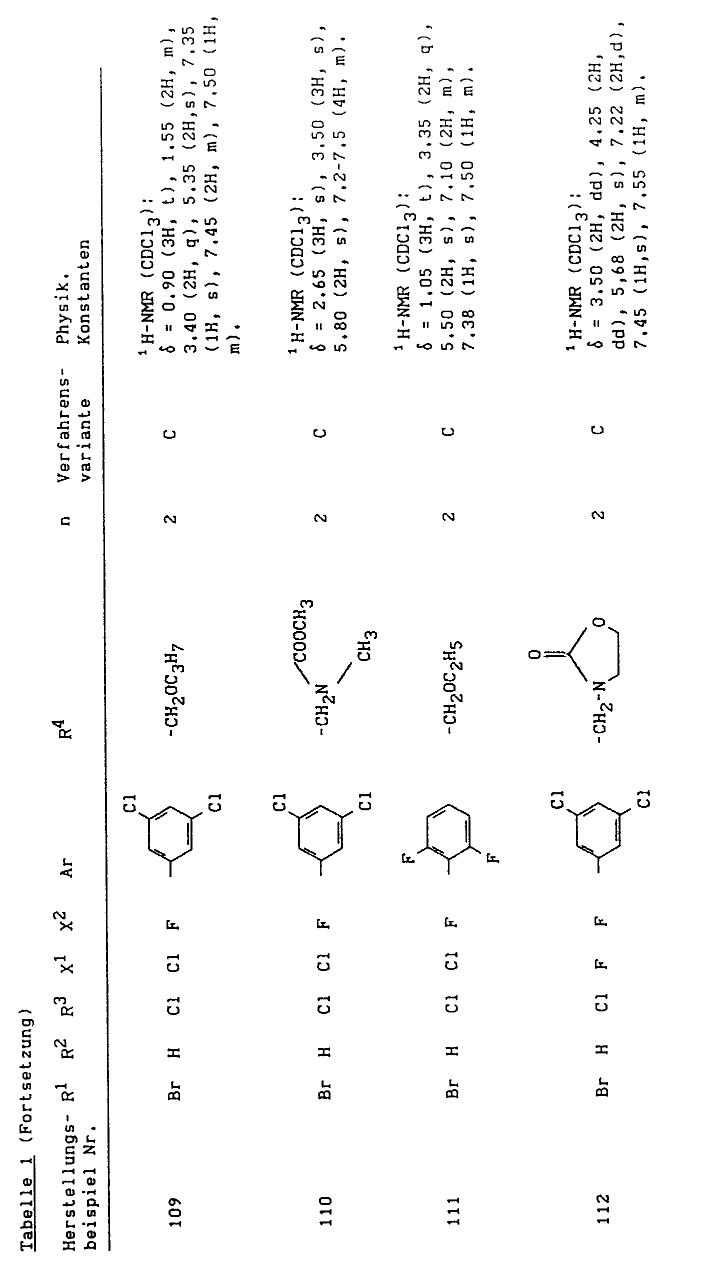

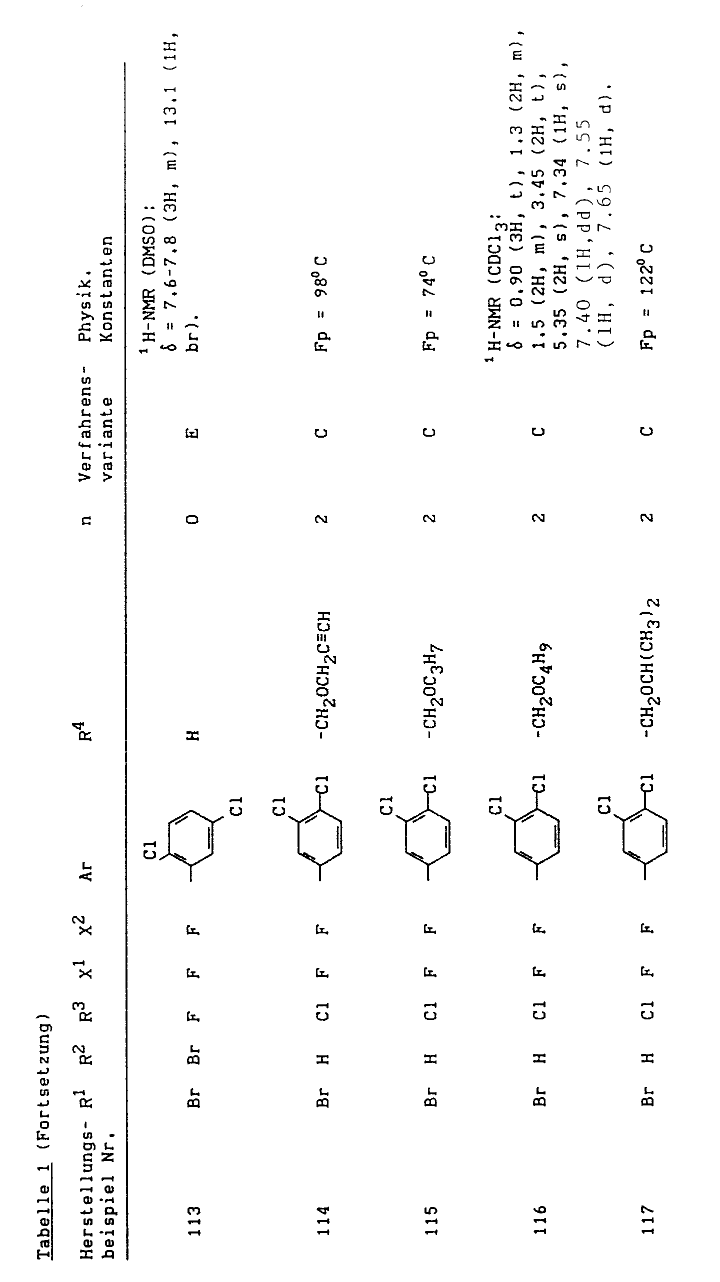

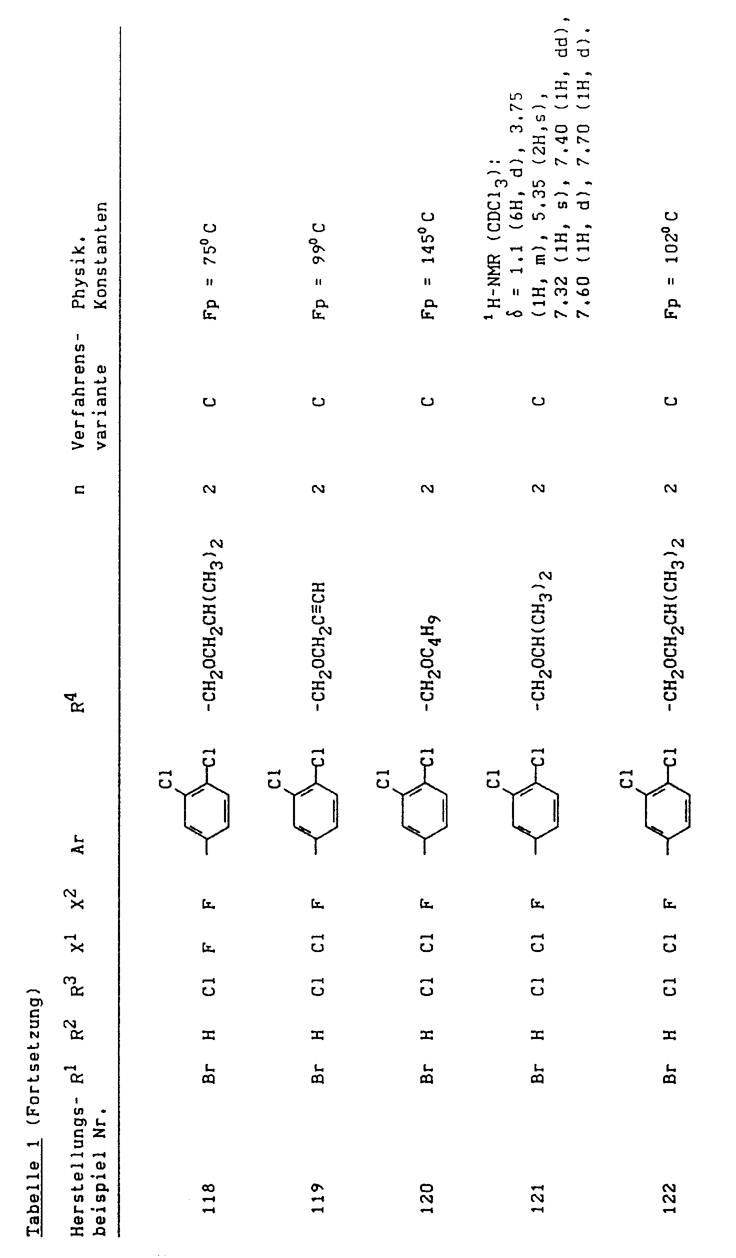

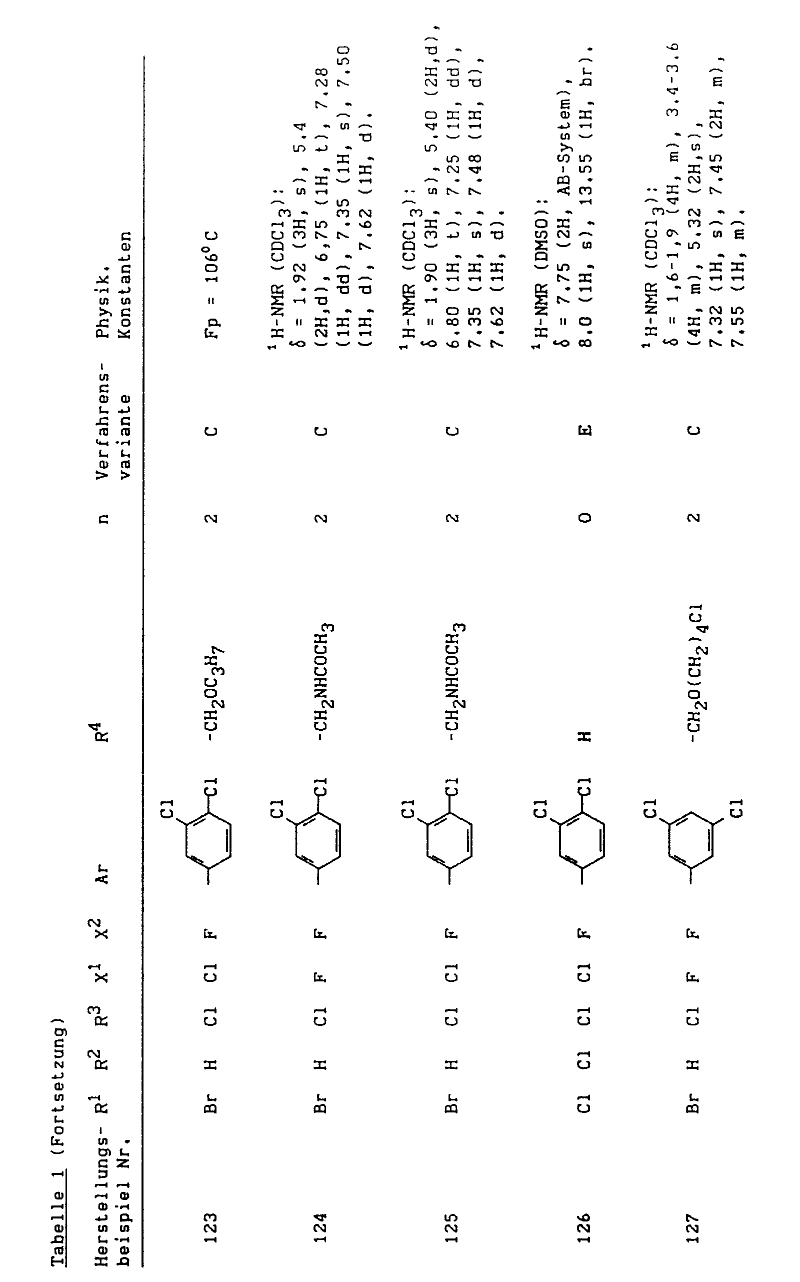

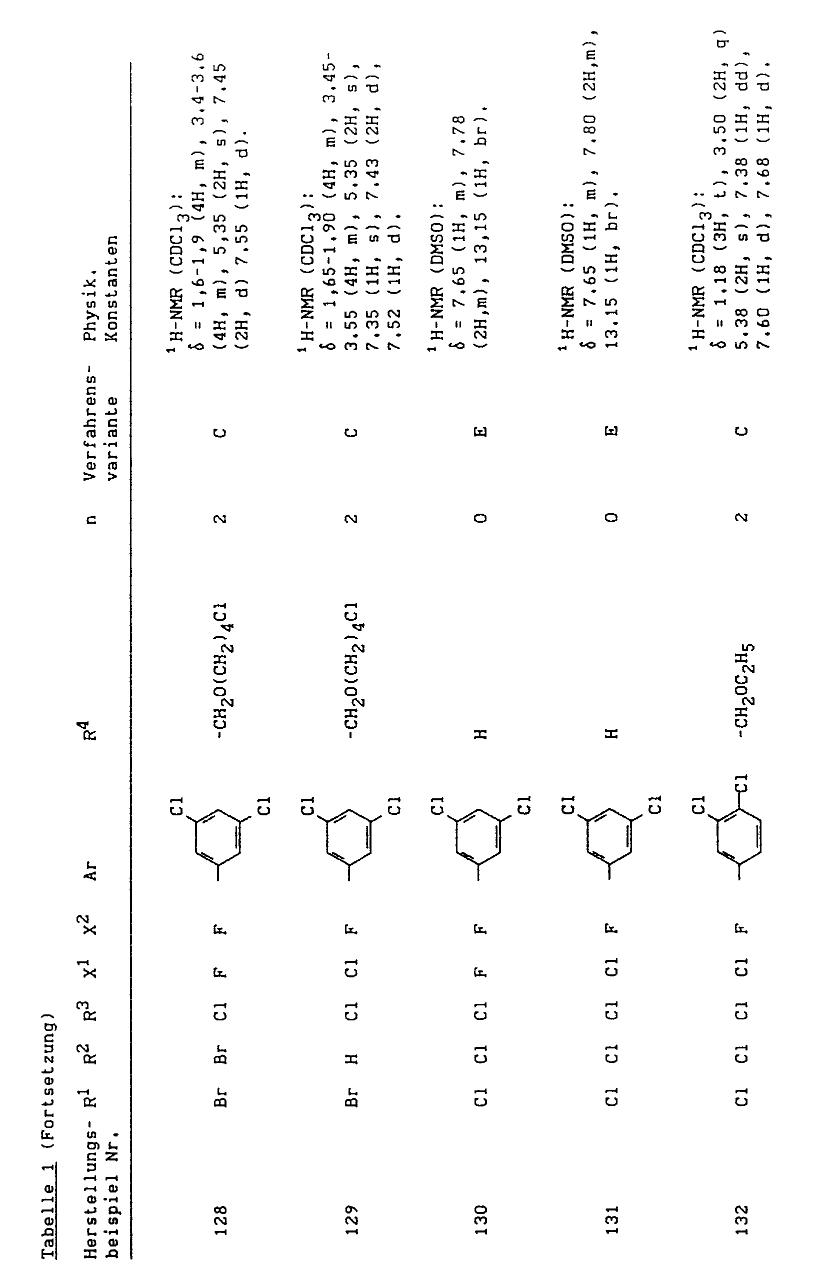

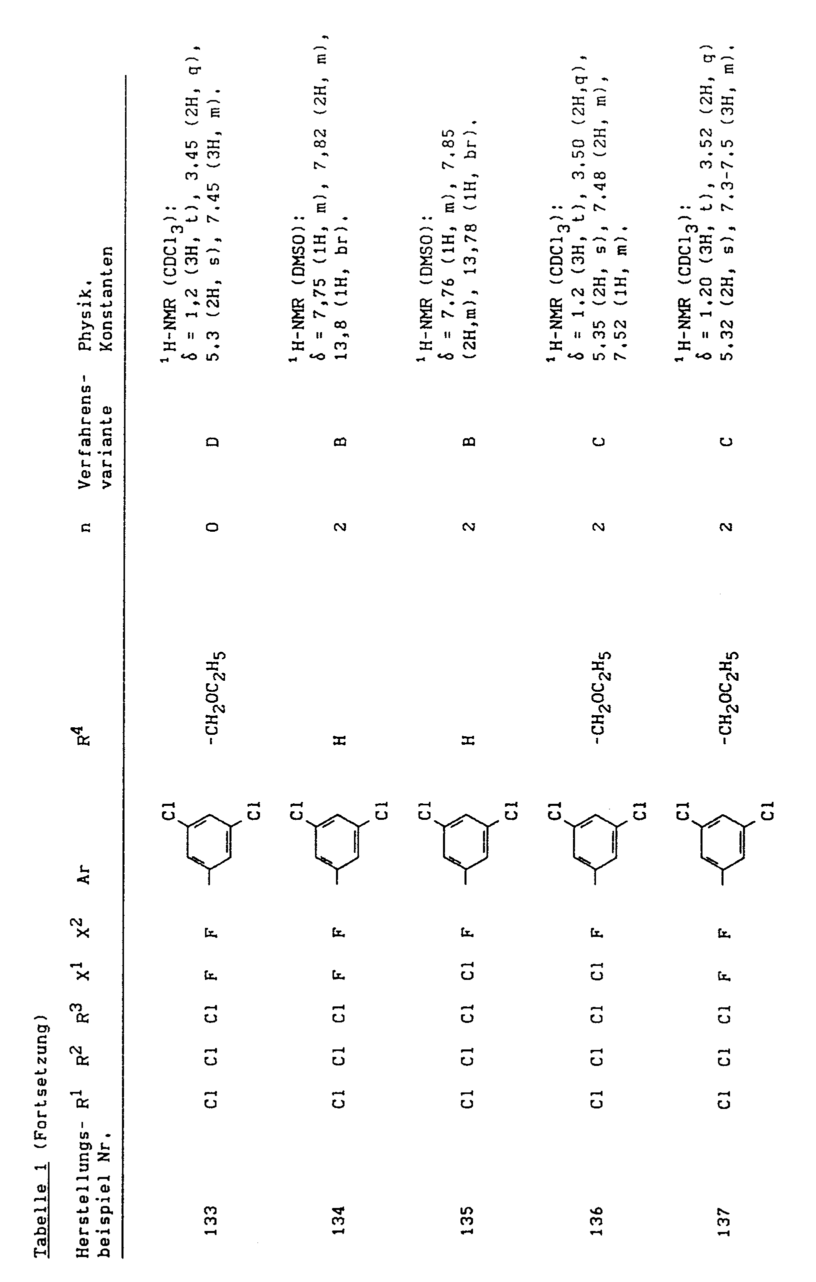

### Herstellung der Vorprodukte

### Beispiel V-1

10 g (0,057 mol) 2-(3-Chlorphenyl)-pyrrol werden in 50 ml trockenem Ether gelöst und mit 6,0 g (0,057 mol) Na₂CO₃ versetzt. Man kühlt auf -20°C und tropft langsam eine Lösung von 6,6 ml (0,0627 mol) Chlordifluormethylsulfenchlorid in 20 ml Ether zu. Man rührt 2,5 h bei dieser Temperatur, filtriert von Festem ab und wäscht mit Ether. Das Filtrat wird mit Wasser gewaschen, über MgSO₄ getrocknet und eingedampft.

Das (tiefblaue) Rohprodukt wird durch Filtrieren über Kieselgel (CH₂CH₂/Petrolether = 1:1) gereinigt.
- Ausbeute:: 10,1 g (= 61 % d. Th.) 2-(3-Chlorphenyl)-5-(chlordifluormethylthio)-pyrrol;
Physikalische Daten siehe Tabelle 2.

In analoger Weise werden auch die in der nachfolgenden Tabelle 2 genannten Vorprodukte der Formel (V) hergestellt.

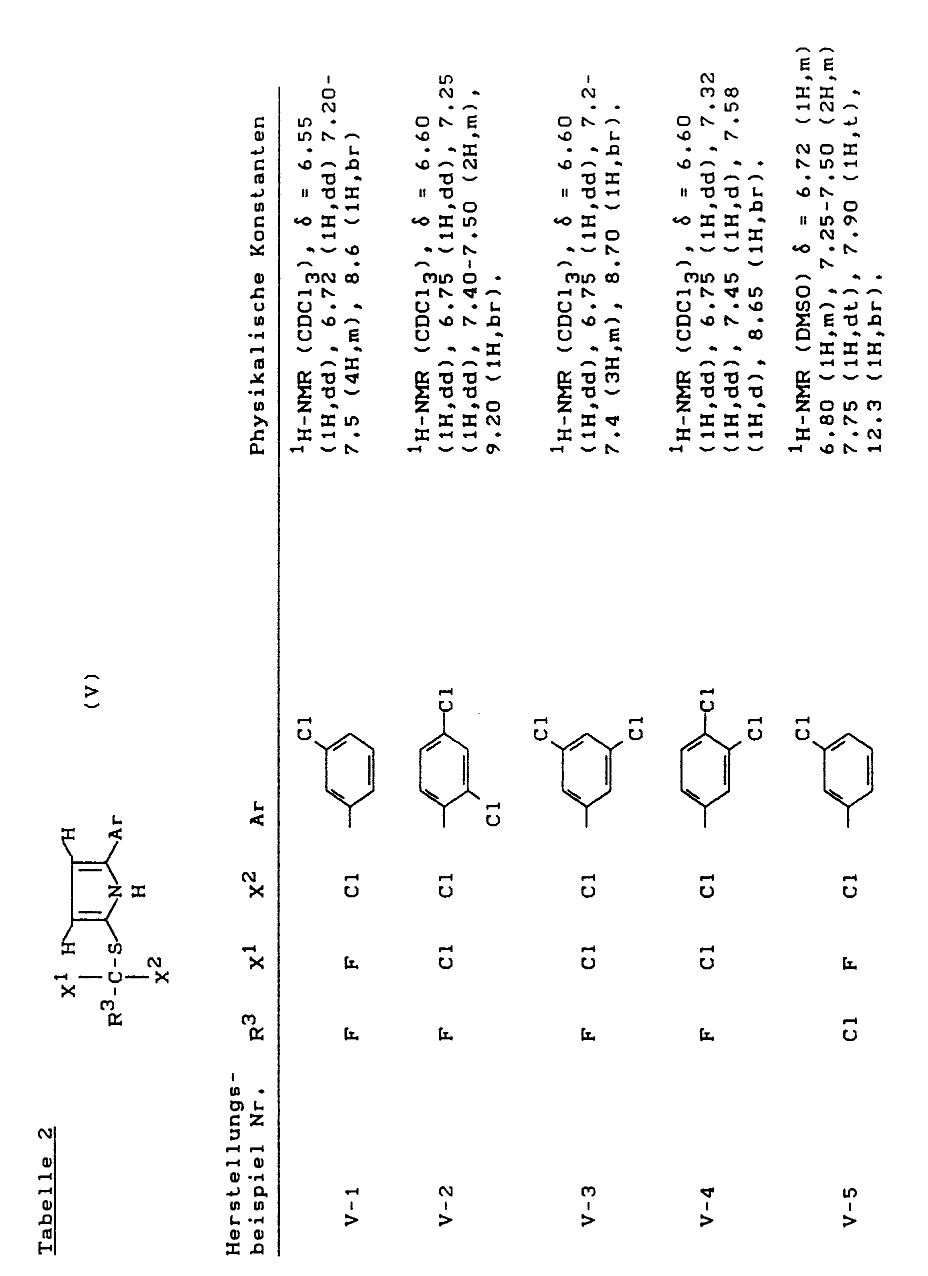

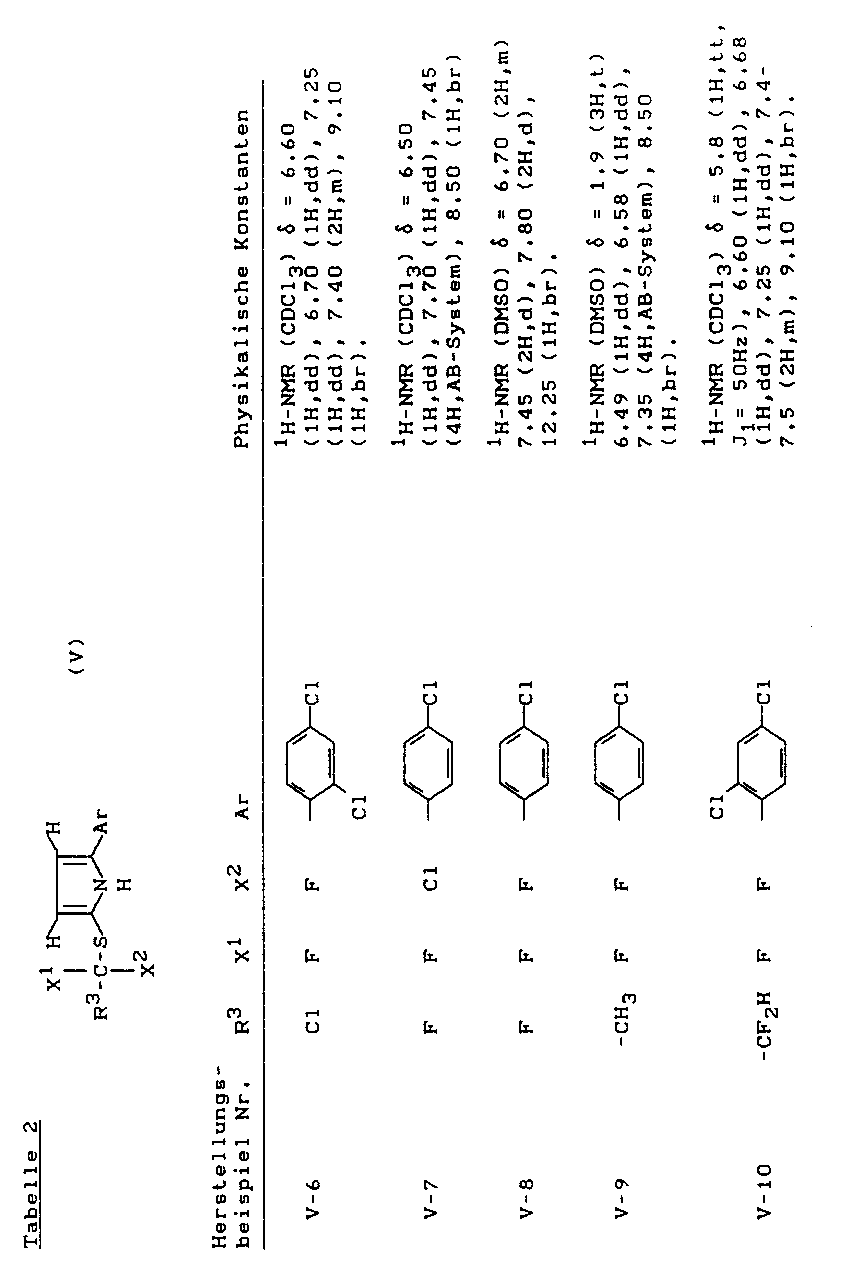

### Beispiel II-1

4,5 g (13,2 mmol) 2-(3-Chlorphenyl)-5-(dichlorfluormethylthio)-pyrrol (V-5) werden in 45 ml Methylenchlorid gelöst und auf -10°C gekühlt. Innerhalb einer Stunde werden 8,29 g (26,4 mmol) 55%ige m-Chlorperbenzoesäure, gelöst in 70 ml CHCl₃ , zugegeben und 3 h bei Raumtemperatur gerührt.

Man wäscht zweimal mit je 50 ml Na₂SO₄-Lösung, zweimal mit je 50 ml Na₂CO₃-Lösung, trocknet über Na₂SO₄ und dampft ein.
- Ausbeute:: 3,96 g (= 91 % d. Th.) 2-(3-Chlorphenyl)-5-(dichlorfluormethylsulfonyl)-pyrrol;
Physikalische Daten siehe Tabelle 3.

In analoger Weise werden auch die in der nachfolgenden Tabelle 3 genannten Vorprodukte der Formel (II) hergestellt.

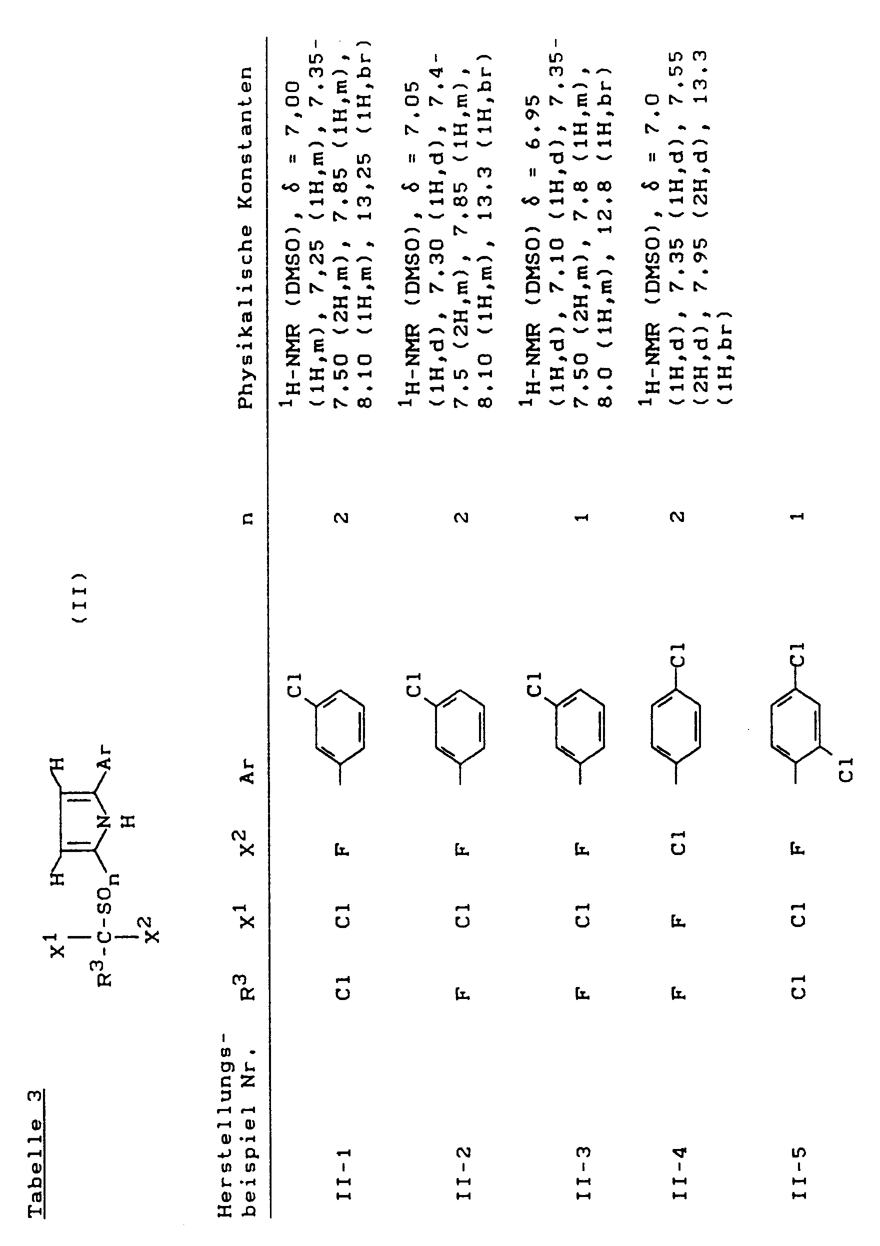

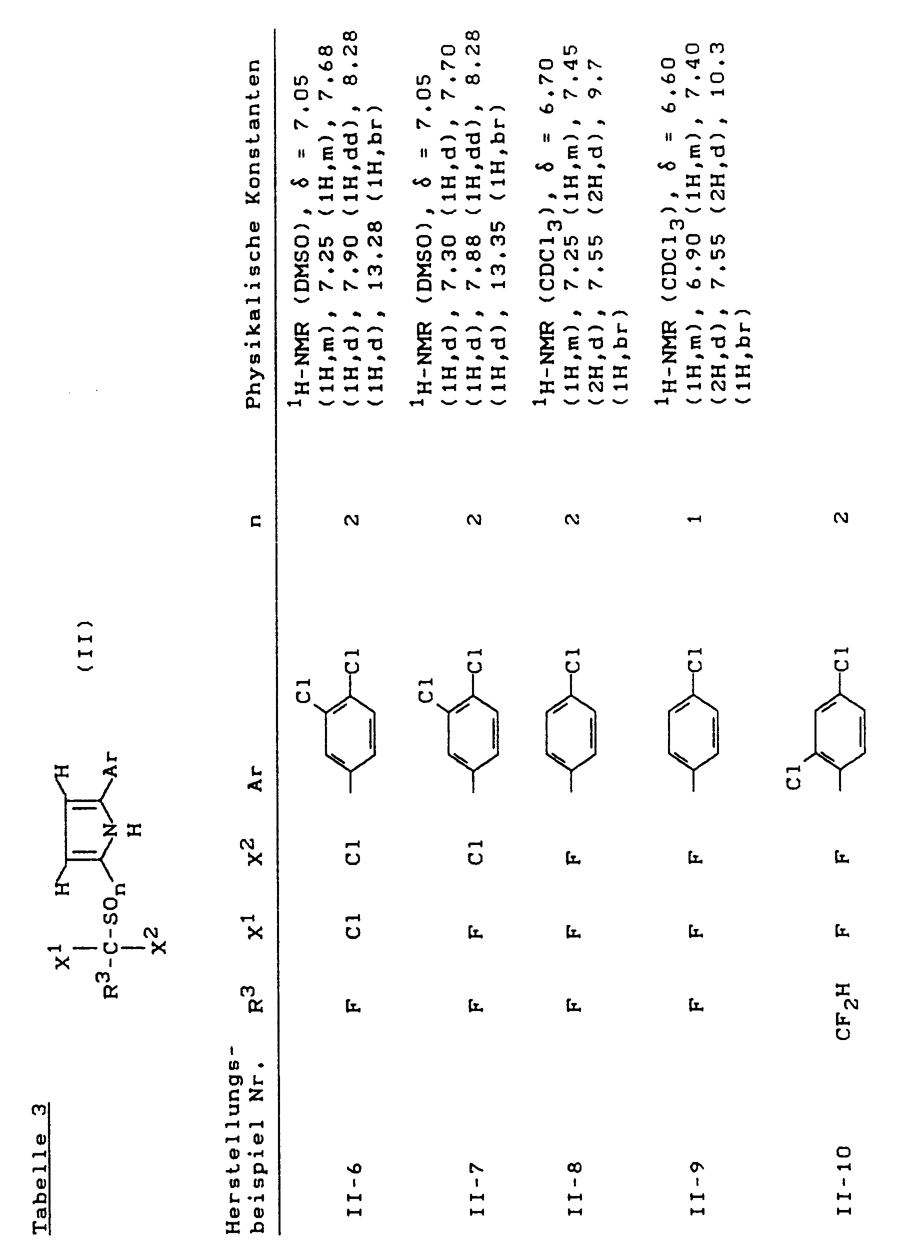

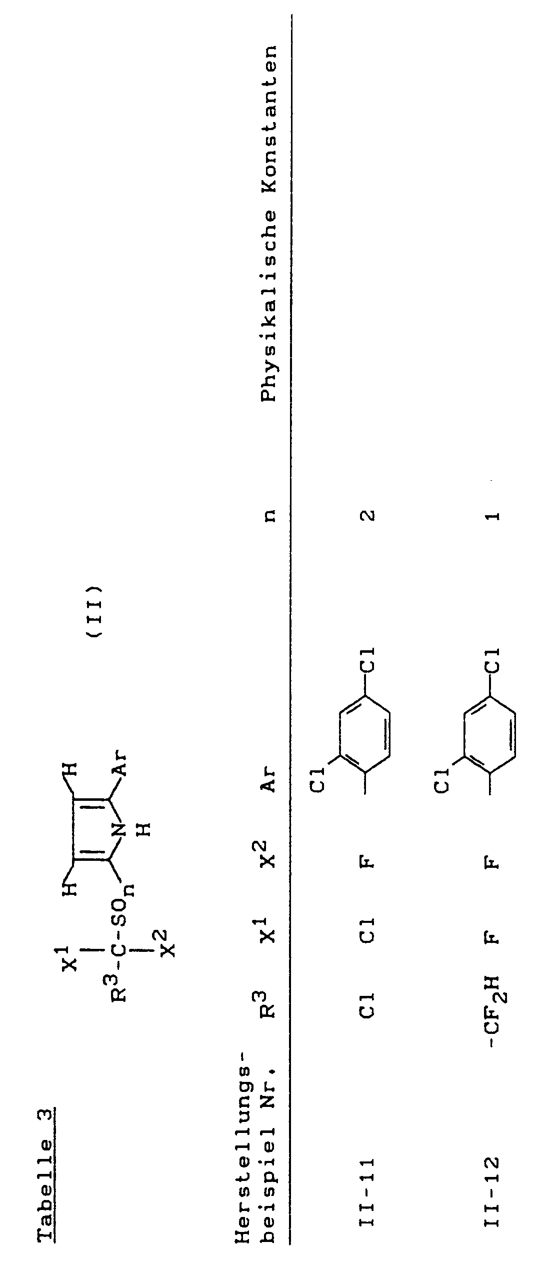

### Anwendungsbeispiele

In den folgenden Anwendungsbeispielen wurde die nachstehend aufgeführte Verbindung aus dem Stand der Technik als Vergleichsverbindung (A) eingesetzt. 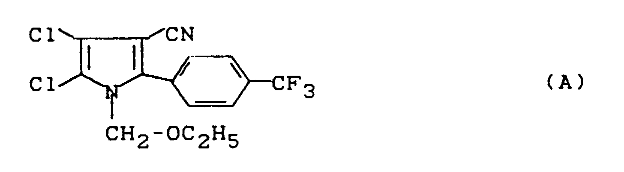
(bekannt aus EP-A 0 347 488; siehe unter Beispiel 9, Seite 27 Zeile 42).

Bei den erfindungsgemäßen Wirkstoffen beziehen sich die Beispiel-Nummern auf die entsprechenden Herstellungsbeispiele.

### Beispiel A

### Plutella-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt, Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: (15), (19), (20), (21), (24), (25), (26), (27), (29), (30), (41).

Nach 7 Tagen zeigt die Vergleichsverbindung (A) einen Abtötungsgrad von 20 %, während die erfindungsgemäßen Wirkstoffe einen Abtötungsgrad von je 100 % zeigen, jeweils bei einer beispielhaften Konzentration von 0,001 %.

### Beispiel B

### Nephotettix-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentrationen behandelt und mit Larven der Grünen Reiszikade (Nephotettix cincticeps) besetzt, solange die Keimlinge noch feucht sind.

Nach der gewünschten Zeit wird die Abtötung in % bestimmt, Dabei bedeutet 100 %, daß alle Zikaden abgetötet wurden; 0 % bedeutet, daß keine Zikaden abgetötet wurden.

Bei diesem Test zeigen z.B. die folgenden Verbindungen der Herstellungsbeispiele überlegene Wirksamkeit gegenüber dem Stand der Technik: (11).

Nach 7 Tagen zeigt die Vergleichsverbindung (A) einen Abtötungsgrad von 20 % und der erfindungsgemäße Wirkstoff von 100 %, jeweils bei einer beispielhaften Konzentrations von 0,01 %.

### Beispiel C

### Tetranychus-Test (OP-resistent)

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschten Konzentrationen.

Bohnenpflanzen (Phaseolus vulgaris), die stark von allen Entwicklungsstadien der gemeinen Spinnmilbe (Tetranychus urticae) befallen sind, werden in eine Wirkstoffzubereitung der gewünschten Konzentration getaucht.

Nach der gewünschten Zeit wird die Wirkung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik: (18), (19), (20), (24), (28), (30), (41).

Nach 7 Tagen zeigen die erfindungsgemäßen Wirkstoffe Abtötungsgrade von 98 bis 100 %, während die Vergleichsverbindung keine Wirkung zeigt, jeweils bei einer beispielhaften Konzentration von 0,1 %.

### Beispiel D

### Fliegentest

- Testtiere:: Musca domestica, Stamm WHO (N)
- Lösungsmittel:: 35 Gewichtsteile Ethylenglykolmonomethylether
- Emulgator:: 35 Gewichtsteile Nonylphenolpolyglykolether

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

2 ml dieser Wirkstoffzubereitung werden auf Filterpapierscheiben (⌀ 9,5 cm) pipettiert, die sich in Petrischalen entsprechender Größe befinden. Nach Trocknung der Filterscheiben werden 25 Testtiere in die Petrischalen überführt und abgedeckt.

Nach 6 Stunden wird die Wirksamkeit der Wirkstoffzubereitung ermittelte. Die Wirksamkeit drückt man in % aus. Dabei bedeutet 100 %, daß alle Fliegen abgetötet wurden; 0 % bedeutet, daß keine Fliegen abgetötet wurden.

Bei diesem Test zeigen z.B. die folgenden Verbindungen der Herstellungsbeispiele überlegene Wirksamkeit gegenüber dem Stand der Technik: (3), (19), (20), (26), (27), (28), (30), (41), (42).

Bei einer beispielhaften Konzentration von 1000 ppm zeigt die Vergleichsverbindung (A) keine Wirkung; dagegen zeigen die genannten erfindungsgemäßen Wirkstoffe hierbei Wirkungen von > 50 %, in einigen Fällen auch von 100 % Abtötung.

### Beispiel E

### Blowfly-Larven-Test

- Testtiere:: Lucilia cuprina-Larven
- Lösungsmittel:: 35 Gewichtsteile Ethylenglykolmonomethylether
- Emulgator:: 35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnt das so erhaltene Emulsionskonzentrat mit Wasser auf die jeweils gewünschte Konzentration.

Etwa 20 Lucilia cuprina res.-Larven werden in ein Teströhrchen gebracht, welches ca. 1 cm³ Pferdefleisch und 0,5 ml der Wirkstoffzubereitung enthält. Nach 24 h wird die Wirksamkeit der Wirkstoffzubereitung ermittelt. Dabei bedeutet 100 %, daß alle Blowfly-Larven abgetötet wurden; 0 % bedeutet, daß keine Blowfly-Larven abgetötet wurden.

In diesem Test zeigen z.B. die folgenden Verbindungen der Herstellungsbeispiele überlegene Wirksamkeit gegenüber dem Stand der Technik: (9), (19), (20), (26), (27), (28), (30).

Bei einer beispielhaften Wirkstoffkonzentration von 1000 ppm zeigen die erfindungsgemäßen Wirkstoffe jeweils einen Abtötungsgrad von 100 %, während die Vergleichsverbindung (A) keine Wirkung zeigt.

### Beispiel F

### Schabentest

- Testtiere:: Blattella germanica oder Periplaneta americana
- Lösungsmittel:: 35 Gewichtsteile Ethylenglykolmonomethylether
- Emulgator:: 35 Gewichtsteile Nonylphenolpolyglykolether

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

2 ml dieser Wirkstoffzubereitung werden auf Filterpapierscheiben (⌀ 9,5 cm) pipettiert, die sich in Petrischalen entsprechender Größe befinden. Nach Trocknung der Filterscheiben werden 5 Testtiere bei B. germanica bzw. P. americana überführt und abgedeckt.

Nach 3 Tagen wird die Wirksamkeit der Wirkstoffzubereitung bestimmt. Die Wirksamkeit drückt man in % aus. Dabei bedeutet 100 %, daß alle Schaben abgetötet wurden; 0 % bedeutet, daß keine Schaben abgetötet wurden.

In diesem Test zeigt z.B. die folgende Verbindung der Herstellungsbeispiele überlegene Wirksamkeit gegenüber dem Stand der Technik: (30).

Bei einer beispielhaften Konzentration von 100 ppm zeigt der erfindungsgemäße Wirkstoff eine Abtötungswirkung von 100 %, während die Vergleichverbindung (A) selbst bei 1000 ppm nur etwa 50 % Wirkung zeigt.

## Patentansprüche

1. Substituierte 2-Arylpyrrole der allgemeinen Formel (I), 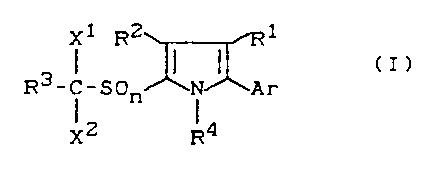 in welcher
R¹ und R² unabhängig voneinander für Wasserstoff oder Halogen stehen, wobei jedoch mindestens einer der beiden Reste R¹ oder R² für Halogen steht,
R³ für Wasserstoff, Halogen oder gegebenenfalls substituiertes Alkyl steht,
R⁴ für Wasserstoff oder 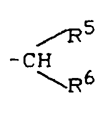 steht,
worin R⁵ für Wasserstoff oder gegebenenfalls substituiertes Alkyl steht und R⁶ für Wasserstoff, gegebenenfalls substituiertes Alkyl oder für einen der Reste 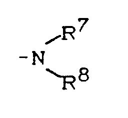 oder -O-R⁷ steht,
worin R⁷ und R⁸ unabhängig voneinander für Wasserstoff, Alkyl, Alkenyl, Alkinyl, Alkoxycarbonyl, Alkenoxycarbonyl, Alkinoxycarbonyl oder Acyl stehen, wobei R⁷ und R⁸ gemeinsam mit dem N-Atom, an welches sie gebunden sind, auch einen Ring bilden können,
Ar für gegebenenfalls substituiertes Aryl steht,
X¹ und X² unabhängig voneinander für Wasserstoff oder Halogen stehen, und
n für 0, 1 oder 2 steht.

2. Substituierte 2-Arylpyrrole der allgemeinen Formel (I) gemäß Anspruch 1, dadurch gekennzeichnet, daß darin
R¹ und R² unabhängig voneinander für Wasserstoff, Brom oder Chlor stehen, wobei mindestens einer der Reste R¹ oder R² für Brom oder Chlor steht,
R³ für Wasserstoff, Fluor, Chlor, Brom oder C₁-C₅-Alkyl steht (welches gegebenenfalls gleich oder ungleich substituiert ist durch 1 bis 5 Fluor-, Chlor- oder Bromatome),
R⁴ für Wasserstoff oder 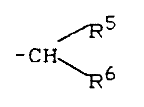 steht,
worin R⁵ für Wasserstoff oder C₁-C₅-Alkyl steht (welches gegebenenfalls substituiert ist durch 1 bis 5 gleiche oder verschiedene Halogenatome, durch C₁-C₅-Alkoxy, C₁-C₅-Alkylthio, C₁-C₅-Acyloxy, C₂-C₆-Alkoxycarbonyl, Phenyl, Cyano oder durch Nitro) und
R⁶ für Wasserstoff oder C₁-C₆-Alkyl steht (welches gegebenenfalls substituiert ist durch 1 bis 5 gleiche oder verschiedene Halogenatome, durch C₁-C₈-Alkoxy, C₁-C₈-Alkylthio, C₁-C₆-Acyloxy, C₂-C₈-Alkoxycarbonyl, Phenyl, Cyano oder durch Nitro) oder worin R⁶ für 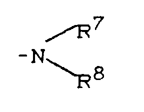 oder -O-R⁷ steht,
wobei R⁷ und R⁸ unabhängig voneinander für Wasserstoff, C₁-C₈-Alkyl, C₃-C₈-Alkenyl oder C₃-C₈-Alkinyl stehen (wobei die Alkyl-, Alkenyl- und Alkinylreste jeweils gegebenenfalls durch 1 bis 6 gleiche oder verschiedene Halogenatome, C₁-C₆-Alkoxy, C₁-C₆-Alkylthio, C₁-C₆-Acyloxy, (C₁-C₆-Alkoxy)-carbonyl, gegebenenfalls substituiertes Phenyl, Cyano oder Nitro substituiert sind), oder für (C₁-C₈-Alkoxy)-carbonyl, (C₃-C₈-Alkenoxy)-carbonyl oder (C₃-C₈-Alkinoxy)-carbonyl stehen (wobei die Alkoxy-, Alkenoxy- und Alkinoxyreste jeweils gegebenenfalls durch 1 bis 6 gleiche oder verschiedene Halogenatome, C₁-C₆-Alkoxy, C₁-C₆-Alkylthio, C₁-C₆-Acyloxy, (C₁-C₆-Alkoxy)-carbonyl, gegebenenfalls substituiertes Phenyl, Cyano oder Nitro substituiert sind), oder für C₁-C₈-Acyl stehen (welches gegebenenfalls durch 1 bis 6 gleiche oder verschiedene Halogenatome, C₁-C₆-Alkoxy, C₁-C₆-Alkylthio, C₁-C₆-Acyloxy, (C₁-C₆-Alkoxy)-carbonyl, gegebenenfalls substituiertes Phenyl, Cyano oder Nitro substituiert ist), oder worin R⁷ und R⁸ zusammen mit dem N-Atom, an welches sie gebunden sind, einen 4-bis 8-gliedrigen Ring bilden,
Ar für Phenyl steht, welches gegebenenfalls ein- bis fünffach gleich oder verschieden substituiert ist durch Halogen,
durch C₁-C₈-Alkyl, C₂-C₈-Alkenyl oder C₂-C₈-Alkinyl (wobei die Alkyl-, Alkenyl- und Alkinylreste jeweils gegebenenfalls durch 1 bis 6 Halogenatome, C₁-C₅-Alkoxy, C₁-C₅-Alkylthio oder durch C₁-C₅-Acyloxy substituiert sind, und wobei die Alkoxy-und Alkylthio-Reste jeweils durch 1-6 Halogenatome substituiert sein können),
durch C₁-C₈-Alkoxy, C₂-C₈-Alkenoxy oder C₂-C₈-Alkinoxy (wobei die Alkoxy-, Alkenoxy- und Alkinoxyreste gegebenenfalls durch 1 bis 6 Halogenatome substituiert sind),
durch C₁-C₈-Alkylthio, C₂-C₈-Alkenylthio oder C₂-C₈-Alkinylthio (wobei die Alkylthio-, Alkenylthio- und Alkinylthio-Reste jeweils gegebenenfalls durch 1 bis 6 Halogenatome substituiert sind),
durch C₂-C₈-Acyloxy (welches gegebenenfalls durch 1 bis 6 Halogenatome substituiert ist),
durch Amino (welches gegebenenfalls substituiert ist durch 1 bis 2 gleiche oder verschiedene Alkylreste oder Halogenalkylreste mit 1 bis 8 Kohlenstoffatomen und gegebenenfalls 1 bis 6 Halogenatomen),
durch Nitro oder Cyano,
und worin
X¹ und X² unabhängig voneinander für Wasserstoff, Fluor, Chlor oder Brom stehen und
n für 0, 1 oder 2 steht.

3. Substituierte 2-Arylpyrrole der allgemeinen Formel (I) gemäß Anspruch 1, dadurch gekennzeichnet, daß darin
R¹ und R² unabhängig voneinander für Wasserstoff, Brom oder Chlor stehen, wobei mindestens einer der beiden Reste R¹ oder R² für Brom oder Chlor steht,
R³ für Wasserstoff, Fluor, Chlor, Brom oder C₁-C₄-Alkyl steht (welches gegebenenfalls gleich oder ungleich substituiert ist durch 1 bis 5 Fluor-, Chlor- oder Bromatome),
R⁴ für Wasserstoff oder 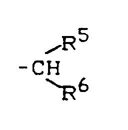 steht,
worin R⁵ für Wasserstoff oder C₁-C₄-Alkyl steht (welches gegebenenfalls substituiert ist durch 1 bis 5 gleiche oder verschiedene Halogenatome, durch C₁-C₄-Alkoxy, C₁-C₄-Alkylthio, C₁-C₄-Acyloxy, C₂-C₅-Alkoxycarbonyl, Phenyl, Cyano oder durch Nitro), und
R⁶ für Wasserstoff oder C₁-C₅-Alkyl steht (welches gegebenenfalls substituiert ist durch 1 bis 5 gleiche oder verschiedene Halogenatome, durch C₁-C₆-Alkoxy, C₁-C₆-Alkylthio, C₁-C₅-Acyloxy, C₂-C₆-Alkoxycarbonyl, Phenyl, Cyano oder durch Nitro), oder worin R⁶ für 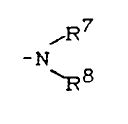 oder -O-R⁷ steht,
worin R⁷ und R⁸ unabhängig voneinander für Wasserstoff, C₁-C₆-Alkyl, C₃-C₆-Alkenyl oder C₃-C₆-Alkinyl stehen (wobei die Alkyl-, Alkenyl- und Alkinylreste jeweils gegebenenfalls durch 1 bis 5 Fluor- und/oder Chloratome, C₁-C₄-Alkoxy, C₁-C₄-Alkylthio, C₁-C₄-Acyloxy, (C₁-C₄-Alkoxy)-carbonyl, gegebenenfalls substituiertes Phenyl, Cyano oder Nitro substituiert sind),
oder für (C₁-C₆-Alkoxy)-carbonyl, (C₃-C₆-Alkenoxy)- oder für (C₃-C₆-Alkinoxy)-carbonyl stehen (wobei die Alkoxy-, Alkenoxy- und Alkinoxyreste jeweils gegebenenfalls durch 1 bis 5 Fluor- und/oder Chloratome, C₁-C₄-Alkoxy, C₁-C₄-Alkylthio, C₁-C₄-Acyloxy, (C₁-C₄-Alkoxy)-carbonyl, gegebenenfalls substituiertes Phenyl, Cyano oder Nitro substituiert sind),
oder für C₁-C₆-Acyl stehen (welches gegebenenfalls durch 1 bis 5 Fluor- und/oder Chloratome, C₁-C₄-Alkoxy, C₁-C₄-Alkylthio, C₁-C₄-Acyloxy, (C₁-C₄-Alkoxy)-carbonyl, gegebenenfalls substituiertes Phenyl, Cyano oder durch Nitro substituiert ist),
oder R⁷ und R⁸ zusammen mit dem N-Atom, an welches sie gebunden sind, über eine beliebige Stelle zu einem 4-bis 6-gliedrigen Ring verknüpft sein können;
in welcher ferner
Ar für Phenyl steht, welches gegebenenfalls einbis vierfach gleich oder verschieden substituiert ist durch Fluor, Chlor oder Brom, oder
durch C₁-C₆-Alkyl, C₂-C₆-Alkenyl oder C₂-C₆-Alkinyl (wobei die Alkyl-, Alkenyl- und Alkinylreste jeweils gegebenenfalls durch 1 bis 5 Fluor- und/oder Chloratome, C₁-C₄-Alkoxy, C₁-C₄-Alkylthio oder durch C₁-C₄-Acyloxy substituiert sind, und wobei die Alkoxy-und Alkylthioreste jeweils durch 1 bis 5 Fluor- und/oder Chloratome substituiert sein können),
durch C₁-C₆-Alkoxy, C₂-C₆-Alkenoxy oder C₂-C₆-Alkinoxy (wobei die Alkoxy-, Alkenoxy- und Alkinoxyreste gegebenenfalls durch 1 bis 5 Fluor- und/oder Chloratome substituiert sind),
durch C₁-C₆-Alkylthio, C₂-C₆-Alkenylthio oder C₂-C₆-Alkinylthio (wobei die Alkylthio-, Alkenylthio- und Alkinylthioreste gegebenenfalls durch 1 bis 5 Fluor-und/oder Chloratome substituiert sind),
durch C₂-C₆-Acyloxy (welches gegebenenfalls durch 1 bis 5 Fluor- und/oder Chloratome substituiert ist),
durch Amino (welches gegebenenfalls substituiert ist durch 1 bis 2 gleiche oder verschiedene Alkylreste mit 1 bis 6 Kohlenstoffatomen, welche durch 1 bis 5 Fluor-und/oder Chloratome substituiert sein können),
durch Nitro oder Cyano,
und worin
X¹ und X² unabhängig voneinander für Wasserstoff, Fluor oder Chlor stehen und
n für 0, 1 oder 2 steht.

4. Verfahren zur Herstellung von substituierten 2-Arylpyrrolen der Formel (I) 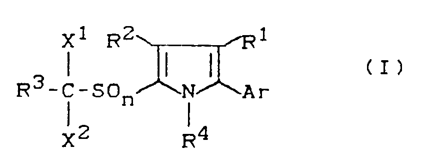 in welcher
R¹ und R² unabhängig voneinander für Wasserstoff oder Halogen stehen, wobei jedoch mindestens einer der beiden Reste R¹ oder R² für Halogen steht,
R³ für Wasserstoff, Halogen oder gegebenenfalls substituiertes Alkyl steht,
R⁴ für Wasserstoff oder 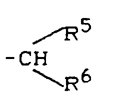 steht,
worin R⁵ für Wasserstoff oder gegebenenfalls substituiertes Alkyl steht und R⁶ für Wasserstoff, gegebenenfalls substituiertes Alkyl oder für einen der Reste 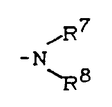 oder -O-R⁷ steht,
worin R⁷ und R⁸ unabhängig voneinander für Wasserstoff, Alkyl, Alkenyl, Alkinyl, Alkoxycarbonyl, Alkenoxycarbonyl, Alkinoxycarbonyl oder Acyl stehen, wobei R⁷ und R⁸ gemeinsam mit dem N-Atom, an welches sie gebunden sind, auch einen Ring bilden können,
Ar für gegebenenfalls substituiertes Aryl steht,
X¹ und X² unabhängig voneinander für Wasserstoff oder Halogen stehen, und
n für 0, 1 oder 2 steht,
dadurch gekennzeichnet, daß man
(A/B) zum Erhalt von 2-Arylpyrrolen der Formel (Ia), 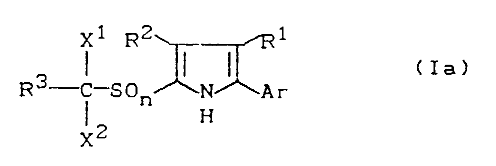 in welcher R¹, R², R³, X¹, X² und Ar die oben angegebene Bedeutung haben und n = 1 oder 2 ist,
entweder
A) 2-Arylpyrrole der Formel (II), 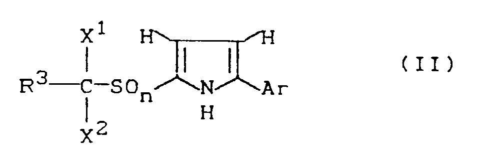
in welcher R³, X¹, X² und Ar die oben angegebenen Bedeutungen haben und n = 1 oder 2 ist,
mit Halogenierungsmitteln umsetzt (Verfahren A)
oder
B) 2-Arylpyrrole der Formel (III) 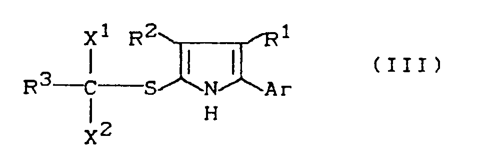
in welcher R¹, R², R³, X¹, X² und Ar die oben angegebenen Bedeutungen haben,
mit Oxidationsmitteln umsetzt (Verfahren B);
oder daß man
C) zum Erhalt von 2-Arylpyrrolen der Formel (Ib), 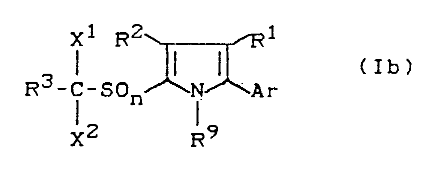
in welcher R¹, R², R³, X¹, X² und Ar die oben angegebene Bedeutung haben und n = 1 oder 2 ist und
R⁹ für 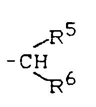 steht, worin R⁵ und R⁶ die oben angegebene Bedeutung haben,
2-Arylpyrrole der allgemeinen Formel (Ia), in welcher R¹, R², R³, X¹, X² und Ar die oben angegebene Bedeutung haben und n = 1 oder 2 ist,
mit Verbindungen der Formel (IV)
R⁹-X³ (IV)
in welcher
R⁹ die oben angegebene Bedeutung hat und
X³ für eine anionische Abgangsgruppe steht,
gegebenenfalls in Gegenwart von Basen und/oder gegebenenfalls in Gegenwart von Verdünnungsmitteln umsetzt (Verfahren C);
oder daß man
D) zum Erhalt von 2-Arylpyrrolen der Formel (Ic) 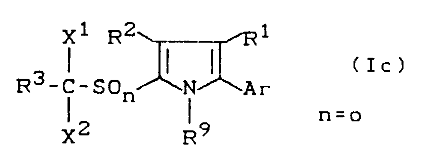
in der R¹, R², R³, X¹, X² und Ar die oben angegebene Bedeutung haben und
R⁹ für 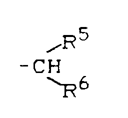 steht, wobei R⁵ und R⁶
die oben angegebene Bedeutung haben und n = 0 ist,
2-Arylpyrrole der allgemeinen Formel (III), in welcher R¹, R², R³, X¹, X² und Ar die oben angegebene Bedeutung haben,
mit Verbindungen der Formel (IV)
R⁹-X³ (IV)
in welcher
R⁹ die oben angegebene Bedeutung hat und
X³ für eine anionische Abgangsgruppe steht,
gegebenenfalls in Gegenwart von Basen und/oder gegebenenfalls in Gegenwart von Verdünnungsmitteln umsetzt (Verfahren D);
oder daß man
E) zum Erhalt von 2-Arylpyrrolen der Formel (Id) 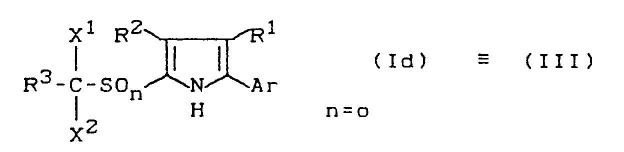
in welcher R¹, R², R³, X¹, X² und Ar die oben angegebene Bedeutung haben und n = 0 ist,
2-Arylpyrrole der Formel (V), 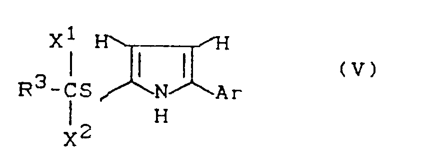 in welcher R³, X¹ , X² und Ar die oben angegebene Bedeutung haben,
mit Halogenierungsmitteln umsetzt (Verfahren E).

5. Schädlingsbekämpfungsmittel, gekennzeichnet durch einen Gehalt an mindestens einem substituierten 2-Arylpyrrol der Formel (I) gemäß Anspruch 1 bis 3.

6. Verwendung von substituierten 2-Arylpyrrolen der Formel (I) gemäß Anspruch 1 bis 3 zur Bekämpfung von tierischen Schädlingen.

7. Verfahren zur Bekämpfung von tierischen Schädlingen, dadurch gekennzeichnet, daß man substituierte 2-Arylpyrrole der Formel (I) gemäß Anspruch 1 bis 3 auf tierische Schädlinge und/oder ihren Lebensraum einwirken läßt.

8. Verfahren zur Herstellung von Schadlingsbekämpfungsmitteln, dadurch gekennzeichnet, daß man substituierte 2-Arylpyrrole der Formel (I) gemäß Anspruch 1 bis 3 mit Streckmitteln und/oder oberflächenaktiven Mitteln vermischt

9. Substituierte 2-Arylpyrrole der allgemeinen Formel (II), 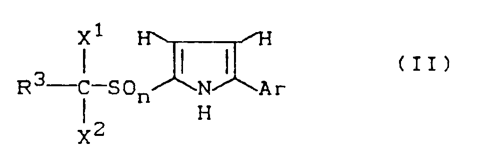 in welcher
n für 1 oder 2 steht,
Ar für gegebenenfalls substituiertes Aryl steht,
R³ für Wasserstoff, Halogen oder gegebenenfalls substituiertes Alkyl steht und
X¹ und X² unabhängig voneinander für Wasserstoff oder Halogen stehen.

10. Substituierte 2-Arylpyrrole der Formel (V), 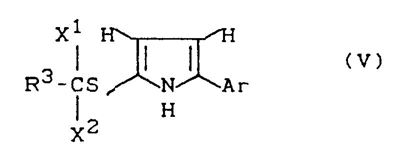 in welcher
Ar, R³, X¹ und X² die in Anspruch 9 genannten Bedeutungen haben.
